# EUROPEAN PATENT SPECIFICATION

(11) **EP 3 516 046 B1**
(45) Date of publication and mention of the grant of the patent: **13.07.2022**
(21) Application number: 17771767.5
(22) Date of filing: 22.09.2017
(51) Int. Cl.: C12N 5/0793, C12N 15/85, C12N 15/861, A61K 35/30, A61K 9/00, A61K 38/00, A61P 27/02, C07K 14/705, C12N 15/86

(54) **OPTOGENETICALLY TRANSFORMED PHOTORECEPTOR PRECURSOR CELLS FOR THE USE IN THE TREATMENT OF RETINAL DEGENERATIVE DISEASES**
OPTOGENETISCH TRANSFORMIERTE PHOTOREZEPTORVORLÄUFERZELLEN ZUR VERWENDUNG IN DER BEHANDLUNG VON DEGENERATIVEN NETZHAUTERKRANKUNGEN
CELLULES DE PRÉCURSEUR DE PHOTORÉCEPTEUR TRANSFORMÉES OPTOGÉNÉTIQUEMENT POUR USAGE DANS LE TRAITEMENT DE MALADIES RÉTINALES DÉGÉNÉRATIVES

(30) Priority: 22.09.2016 EP 16306225
(43) Date of publication of application: 31.07.2019
(73) Proprietor: Sorbonne Université, 75006 Paris (FR); Centre National de la Recherche Scientifique, 75016 Paris (FR); INSERM (Institut National de la Santé et de la Recherche Médicale), 75013 Paris (FR); Technische Universität Dresden (TU Dresden), 01069 Dresden (DE)
(72) Inventor: DUEBEL, Jens, 75005 Paris (FR); ADER, Marius, 01309 Dresden (DE); CHAFFIOL, Antoine, 91220 Brétigny sur Orge (FR); GARITA-HERNANDEZ, Marcela, 75012 Paris (FR); LAMPIC, Marusa, 75010 Paris (FR); DALKARA, Deniz, 94140 Alfortville (FR); GOUREAU, Olivier, 75012 Paris (FR); SAHEL, José-Alain, 75013 Paris (FR)
(74) Representative: Cabinet Becker et Associés
(86) International application number: PCT/EP2017/074125
(87) International publication number: WO 2018/055131

(56) References cited:
- WO-A1-2016/097183
- WO-A1-2016/135457
- H. CHENG ET AL: "Excess cones in the retinal degeneration rd7 mouse, caused by the loss of function of orphan nuclear receptor Nr2e3, originate from early-born photoreceptor precursors", HUMAN MOLECULAR GENETICS, vol. 20, no. 21, 3 August 2011 (2011-08-03), pages 4102-4115, XP055335981, gb ISSN: 0964-6906, DOI: 10.1093/hmg/ddr334
- ALI AHMED M ET AL: "Optogenetic Inhibitor of the Transcription Factor CREB", CHEMISTRY AND BIOLOGY, vol. 22, no. 11, 19 November 2015 (2015-11-19), pages 1531-1539, XP029306638, ISSN: 1074-5521, DOI: 10.1016/J.CHEMBIOL.2015.09.018
- ALONA O. BARNEA-CRAMER ET AL: "Function of human pluripotent stem cell-derived photoreceptor progenitors in blind mice", SCIENTIFIC REPORTS, vol. 6, 13 July 2016 (2016-07-13), page 29784, XP055305236, DOI: 10.1038/srep29784
- MEAD BEN ET AL: "Stem cell treatment of degenerative eye disease", STEM CELL RESEARCH, vol. 14, no. 3, 24 February 2015 (2015-02-24), pages 243-257, XP029171995, ISSN: 1873-5061, DOI: 10.1016/J.SCR.2015.02.003
- J.-J. PANG ET AL: "AAV-Mediated Gene Therapy in Mouse Models of Recessive Retinal Degeneration", CURRENT MOLECULAR MEDICINE, vol. 12, no. 3, 1 March 2012 (2012-03-01), pages 316-330, XP055335865, NL ISSN: 1566-5240, DOI: 10.2174/156652412799218877

## Description

### Field of the invention

The present invention relates to the field of the medicine, in particular to the treatment of retinal degenerative diseases.

### Background of the invention

Retinal degenerative diseases leading to the loss of photoreceptors are a major cause of untreatable blindness in the developed world. Possible strategies for late-stage retinal rescue include electrical retinal implants as well as new approaches, such as cell replacement therapy (stem cell approaches) or optogenetics.

Cell replacement therapy, using photoreceptor precursor cells, allows substitution for degenerated photoreceptors in blinding diseases, such as retinitis pigmentosa or age related macular degeneration. However, a key problem of cell replacement therapy is that transplanted photoreceptors have to develop into healthy photoreceptors with light sensitive outer segments. Barber et al. showed that while in wild-type mice and mouse models of slow retinal degeneration (Gnat1^{-/-}) restoration visual function after photoreceptor transplantation has been shown, cell replacement strategies have been more challenging in models of fast and severe degeneration (e.g. PDE6βrd1/rd1) (Barber AC et al. Proc Natl Acad Sci USA. 2013 Jan 2;110(1):354-9). In these animal models, transplanted precursor cells are not able to integrate well in the existing diseased retina and do not develop into normal healthy photoreceptors with light sensitive outer segments and as such, do not seem to be functioning.

The ability of immature photoreceptor precursors to elaborate functional outer segments depends significantly on the physiological state, in particular glial scarring and changes in the outer layer membrane (OLM) of the diseased retina of the recipient (Barber AC et al. Proc Natl Acad Sci USA. 2013 Jan 2;110(1):354-9). The development and the maintenance of light sensitive outer segments is a very complex process that requires a tight contact between the photoreceptor outer segments and the retinal pigment epithelium (RPE). Importantly, the RPE supplies nutrients to photoreceptors and it is important for the renewal of outer segments (disc shedding). Finally, the RPE is essential for conversion of all-trans retinol to 11-cis retinal, the chromophore of the visual pigments and is thus critical for the visual cycle (chromophore recycling).

Alternatively, optogenetic tools are also used to restore visual function by conferring light sensitivity to cells of the inner retina. Genetically encoded light sensitive proteins are introduced into bipolar or retinal ganglion cells or 'dormant' cones via viral vectors. This approach is nevertheless limited, as it can only rescue the function of remaining cells, but it cannot renew degenerated neural structures.

Accordingly, there is a significant need for an improved strategy to treat retinal degenerative diseases leading to the loss of photoreceptors, in particular in patients with advanced stages of disease.

### Summary of the invention

The inventors have shown that the treatment of retinal degenerative diseases can be greatly improved, in particular for patients with advanced stages of disease, by combining the cell replacement approach with an optogenetic approach. The strategy is to introduce an optogenetic inhibitor (e.g. Halorhodopsin or Jaws) in photoreceptor precursors prior to transplantation. Illumination of cells expressing this optogenetic tool generates hyperpolarization, thus mimicking the function of healthy photoreceptors to light. The main advantage of this approach is that the integrated photoreceptors not only restore visual function via the optogenetic inhibitor, even without correctly developed outer segments, but also provide a renewal of neural degenerated structures. Moreover, these optogenetically transformed photoreceptors do not require the cycling of the retinoid analogs between the RPE and photoreceptor cells.

Accordingly, in a first aspect, the present invention relates to isolated photoreceptor precursor cells, comprising a heterologous nucleic acid encoding an optogenetic inhibitor as defined in the claims.

Preferably, said nucleic acid is operably linked to a specific-photoreceptor promoter.

Preferably, said heterologous nucleic acid is contained in a recombinant viral vector, more preferably an adeno-associated virus.

The optogenetic inhibitor is selected from the group consisting of halorhodopsins, archaerhodopsin-3 (AR-3), archaerhodopsin (Arch), bacteriorhodopsins, proteorhodopsins, xanthorhodopsins, Leptosphaeria maculans fungal opsins (Mac) and Jaws, more preferably selected from halorhodopsins and Jaws.

Preferably, said photoreceptor precursor cells were obtained from differentiation of stem cells, more preferably from induced pluripotent stem cells.

In a second aspect, the present invention relates to a pharmaceutical composition comprising photoreceptor precursor cells of the invention and a pharmaceutically acceptable excipient. Preferably, said composition is formulated for intraocular injection.

In a third aspect, the present invention relates to photoreceptor precursor cells or pharmaceutical composition of the invention for use in the treatment of a retinal degenerative disease, preferably a retinal degenerative disease related to a loss of function or death of photoreceptors.

Preferably, said cells or composition are to be administered to the subject in need thereof by intraocular injection, more preferably by injection into the subretinal space of the eye.

Preferably, the retinal degenerative disease is selected from the group consisting of macular degeneration, retinitis pigmentosa, cone dystrophy, Usher syndrome, rod dystrophy, rod-cone dystrophy, achromatopsia and Bardet-Biedl syndrome.

Preferably, the patient to be treated with photoreceptor precursor cells or pharmaceutical composition of the invention is a patient with advanced stage of photoreceptor degeneration.

In a last aspect, the present invention relates to an *in vitro* method for producing the photoreceptor precursor cells of the invention, comprising introducing a nucleic acid encoding optogenetic inhibitor as defined the claims into provided photoreceptor precursor cells.

Preferably, the provided photoreceptor precursor cells provided were obtained from differentiation of stem cells, more preferably from differentiation of adult stem cells or induced pluripotent stem cells. In particular, provided photoreceptor precursor cells may have been obtained from induced pluripotent stem cells obtained from somatic cells, e.g. fibroblasts, from a patient suffering from a retinal generative disease.

### Brief description of the drawings.

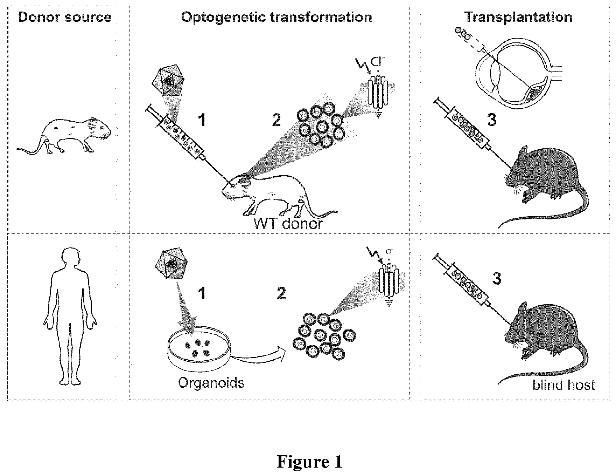

**Figure 1****.** Schematic presentation of optogenetically transformed photoreceptors, derived from mice (top) or hiPSCs (bottom). Top: Eyes of new-born wild-type mice (P2) were injected with a viral vector encoding NpHR under the control of rhodopsin promoter (AAV-hRho-NpHR-GFP). At P4, retinas were dissected and photoreceptor precursors were sorted out by using a photoreceptor specific marker. These harvested cells were then transplanted via sub-retinal injections into blind mice. Bottom: Human iPSCs were differentiated towards retinal organoids. Retinal organoids were infected with a viral vector carrying Jaws gene under the control of cone arrestin promoter (AAV-mCar-Jaws-GFP). After further maturation, cells were dissociated and iPSC-derived photoreceptors were transplanted via sub-retinal injections into blind mice.

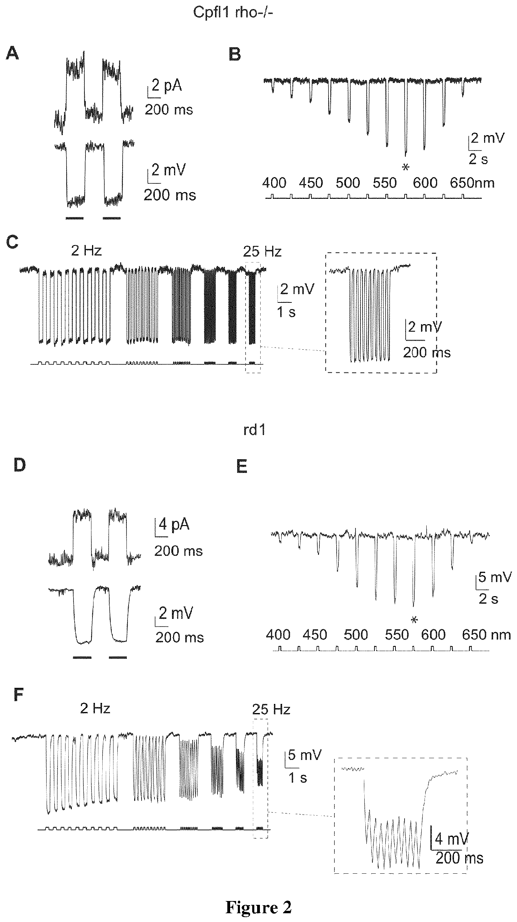

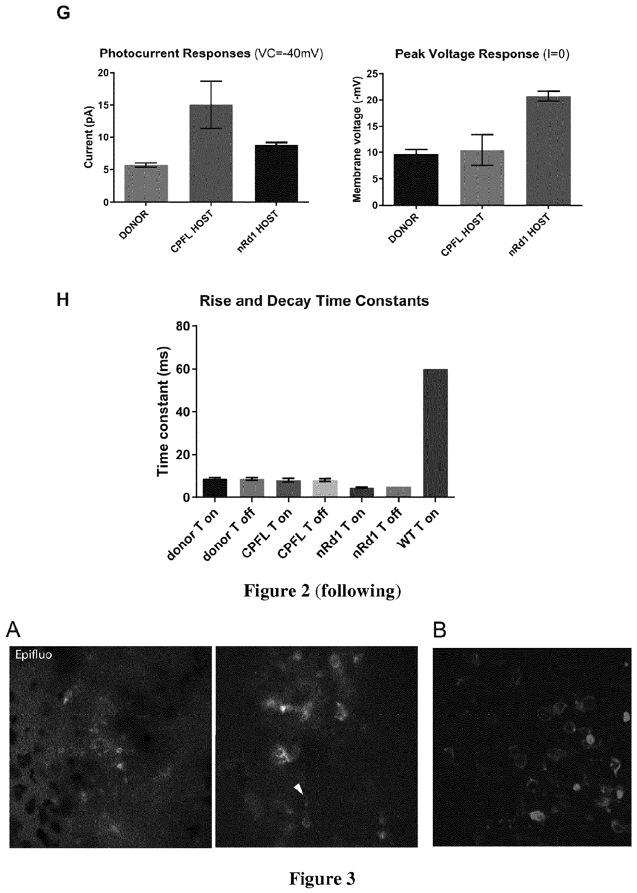

**Figure 2****.** Integrated transplanted photoreceptor precursors, expressing halorhodopsin, respond to light. (A-D) *Cpfl1*/*Rho*^{*-*/*-*} model, (E-G) *rd1* model. (A, E) Light-evoked responses of NpHR-expressing photoreceptor precursors stimulated with 2 consecutive flashes of light at 590 nm at an intensity of 1 × 10¹⁶ photons cm⁻² s⁻¹ (top, current response; bottom, voltage response). (B, F) Photocurrent action spectrum corresponding to a NpHR-expressing cell stimulated at different wavelengths. Stimuli ranging from 400 nm to 650 nm, separated by 25 nm steps, were used at 3.5 × 10¹⁷ photons cm⁻² s⁻¹. Maximal responses were obtained at 575 nm. (C) Same as in (B) but with continuous 'rainbow' stimulation between 350 and 680 nm. (D, G)Temporal properties: Modulation of NpHR-induced photocurrents at increasing stimulation frequencies (from 2 to 25 Hz; at 3.5 × 10¹⁷ photons cm⁻² s⁻¹), a magnified trace is shown for 25 Hz. (H) Comparison of light response characteristics between NpHR-photoreceptor precursors transplanted Cpfl1/Rho^{-/-} and rd1 models. (K) Comparison of response amplitude. Mean photocurrent peak (top left) obtained at -40mV in voltage-clamp configuration, or mean peak voltage response (top right) obtained in current-clamp configuration (current zero). Comparison of rise and decay time constants between transplanted cells in the two models (in current clamp 'zero' configuration) at 590 nm and 1 × 10¹⁶ photons cm⁻² s⁻¹ (bottom).
**Figure 3****:** Live imaging of GFP-positive transplanted cells in the isolated retina of the CPFL mouse model. (A) Epifluorescence images of transplanted photoreceptor cells 20 days after injection in a whole-mount retina (photoreceptors side-up). The white arrowhead shows a transplanted cell with a putative spherule (synaptic output). (B) Live 2-photon image showing the density of the transplanted cells, optogene expression was restricted to PR-membranes.

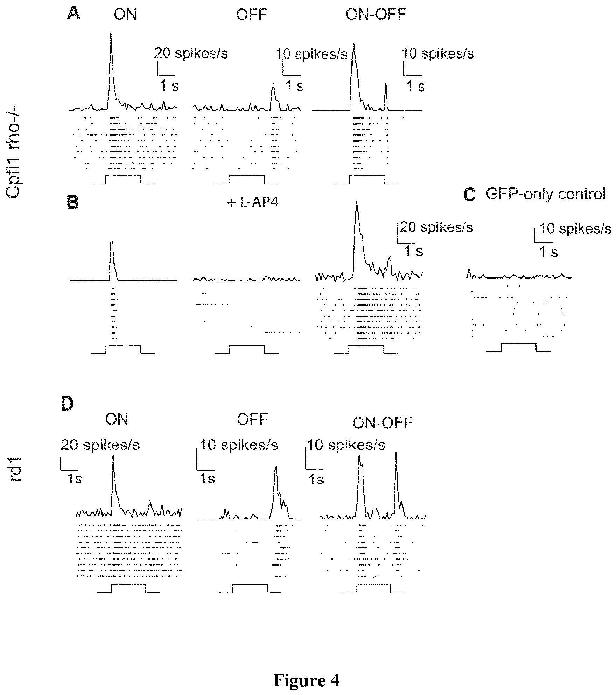

**Figure 4****:** Halorhodopsin-triggered responses from transplanted photoreceptors are transmitted to retinal ganglion cells. (A-C) Averaged spike responses obtained from multi-electrode array (MEA) recordings shown as PSTH (peristimulus time histogram) and raster plots recorded in transplanted Cpfl1/Rho^{-/-} mice (580 nm; 1.24 × 10¹⁷ photons cm⁻² s⁻¹). (A) Representative traces from three RGCs responding either with an ON-response (left), OFF-response (center) or an ON/OFF-response (right). (B) Representative PSTH and raster plots from a cell showing ON-response before (left) and during perfusion with L-AP4. Application of L-AP4 specifically blocks the ON bipolar cell response as shown in the center compared to a response after wash-out shown on the right. PSTHs are presented as mean calculated for a single cell over 10 repetitions, raster plots of the same cells are shown below. (C) Representative PSTH and raster plots recorded from an unresponsive cell from a control retina transplanted with GFP-only-expressing photoreceptor precursor cells. (D) Averaged spike responses obtained from multi-electrode array (MEA) recordings shown as PSTH (peristimulus time histogram) and raster plots recorded in transplanted rd1 mice (580 nm; 1.24 × 10¹⁷ photons cm⁻²s⁻¹)-representative traces from three RGCs responding either with an ON-response (left), OFF-response (center) or an ON/OFF-response (right).

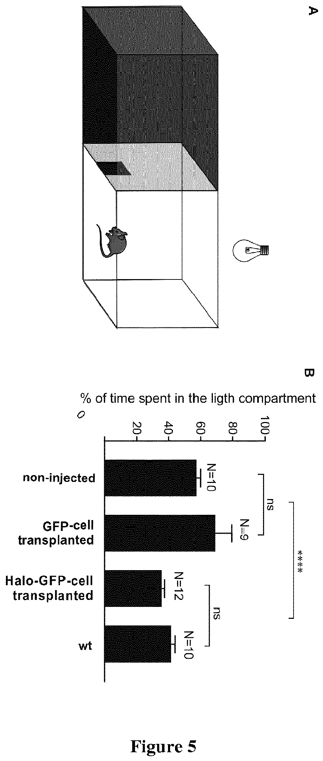

**Figure 5****:** Halorhodopsin-triggered responses from transplanted photoreceptors induce light avoidance behavior in blind mice. (A) Schematic representation of the light/dark box test used to measure light avoidance behaviour in mice. (B) Percentage of time spent in the light compartment. Control groups: non-injected Cpfl1/Rho^{-/-} mice, GFP-expressing cells transplanted, N = 9; Halorhodopsin group: NpHR-YFP-expressing cells transplanted, N = 12; Wt group: non-injected wt mice. Mean ± SEM are shown for each group. An illumination intensity of 2.11 × 10¹⁵ photons cm⁻² s⁻¹ was used.

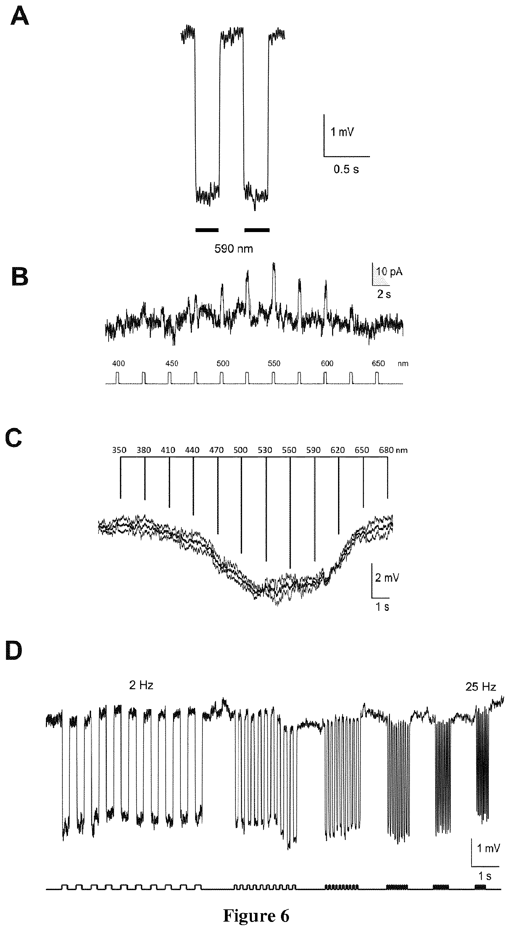

**Figure 6****:** Optogenetic stimulation triggers Halorhodopsin-induced light responses in transplanted photoreceptors of blind *rd10* mice. (A) Representative examples of membrane potential hyperpolarization of a Halorhodopsin-expressing cell stimulated with 2 consecutive flashes of light at 590 nm and an intensity of 1.3 10¹⁶ photons cm⁻² s⁻¹. (B) Halorhodopsin-induced photocurrents as a function of stimulation wavelength. Stimuli ranging from 400 nm and 650 nm, separated by 25 nm steps, were used (at 1.3 10¹⁶ photons cm⁻² s⁻¹). Maximal responses were obtained at 550-575 nm. (C) Same stimulus as in (B) but with continuous 'rainbow' stimulation between 350 and 680 nm (current-clamp). (D) Temporal properties: modulation of Halorhodopsin-induced membrane hyperpolarization at increasing stimulation frequencies (from 2 to 25Hz; at 1.3×10¹⁶ photons cm⁻² s⁻¹). All traces from A to D were obtained using the whole-cell patch-clamp recording technique.

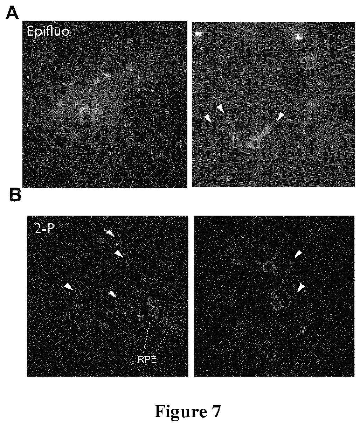

**Figure 7****:** Live imaging of GFP-positive transplanted cells in the isolated retina of the *rd10* mouse model. (A) Epifluorescence images of transplanted photoreceptor cells 20 days after injection in a whole-mount retina (photoreceptors side-up). The white arrowheads (right) show transplanted cell with a putative spherule (synaptic output). (B) Live 2-photon images showing the density of the transplanted cells, and that optogene expression is restricted to PR-membranes (left, arrowheads). Some transplanted cells displayed nice prolongations (right, arrowheads).

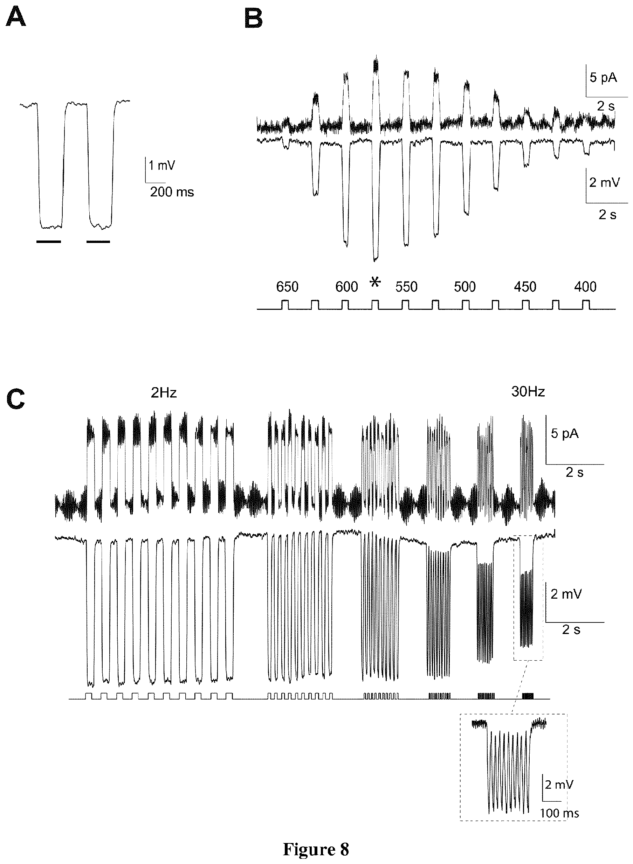

**Figure 8****:** Optogenetic stimulation triggers Halorhodopsin-induced light responses in donor cells. (A) Representative example of membrane potential hyperpolarization of a Halorhodopsin-expressing cell stimulated with 2 consecutive flashes of light at 590 nm and an intensity of 1.3 10¹⁶ photons cm⁻² s⁻¹. (B) Halorhodopsin-induced photocurrents (top) or membrane hyperpolarization (bottom) as a function of stimulation wavelength. Stimuli ranging from 400 nm and 650 nm, separated by 25 nm steps, were used (at 1.3 10¹⁶ photons cm⁻² s⁻¹). Maximal responses were obtained at 575 nm. (C) Modulation of Halorhodopsin-induced photocurrents (top) and membrane hyperpolarization (bottom) at increasing stimulation frequencies (from 2 to 25 Hz; at 1.3×10¹⁶ photons cm⁻² s⁻¹). A magnified trace is shown for 25 Hz. All traces from A to C were obtained using the whole-cell patch-clamp recording technique.

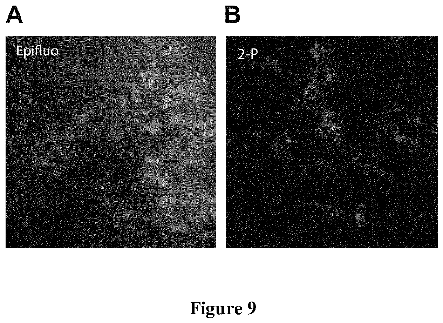

**Figure 9****:** Live imaging of GFP-positive cells in the isolated retina of the *donor.* (A) Epifluorescence image of photoreceptor cells 20 days after AAV-injection in a whole-mount retina (photoreceptors side-up). Fluorescence was restricted to the cell membrane. (B) Live 2-photon images showing the high density of fluorescent donor cells, and that optogene expression is restricted to PR-membranes.

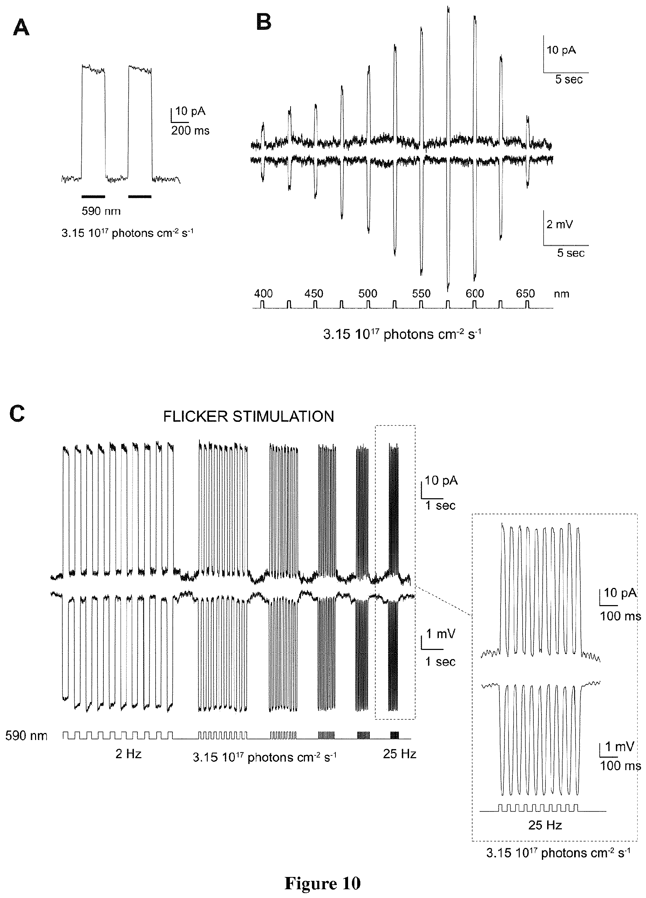

**Figure 10****:** Optogenetic stimulation triggers light responses in AAV-transduced hiPS cells expressing Jaws in retinal organoids. (A) Cell typical light responses: representative examples of photocurrents from a Jaws-expressing cell stimulated with 2 consecutive flashes of light at 590 nm at an intensity of 3.5 10¹⁷ photons cm⁻² s⁻¹. (B) Jaws-induced photocurrents (top) and hyperpolarizations (bottom) as a function of stimulation wavelength. Stimuli ranging from 400 nm and 650 nm, separated by 25 nm steps, were used (at 1.3 10¹⁶ photons cm⁻² s⁻¹). Maximal responses were obtained at 575 nm. (C) Temporal properties: modulation of Jaws-induced membrane photocurrents (top) and hyperpolarization (bottom) at increasing stimulation frequencies (from 2 to 25Hz; at 3.5×10¹⁷ photons cm⁻² s⁻¹), a magnified trace is shown for 25 Hz. All traces from B to D were obtained using the whole-cell patch-clamp recording technique.

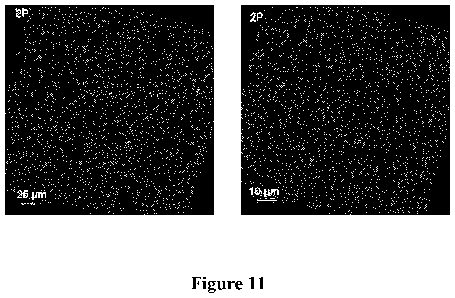

**Figure 11****:** Live imaging of GFP-positive cells in a retinal organoid at different magnification. The fluorescence observed indicates that Jaws expression is restricted to PR-membranes (live 2-photon images).

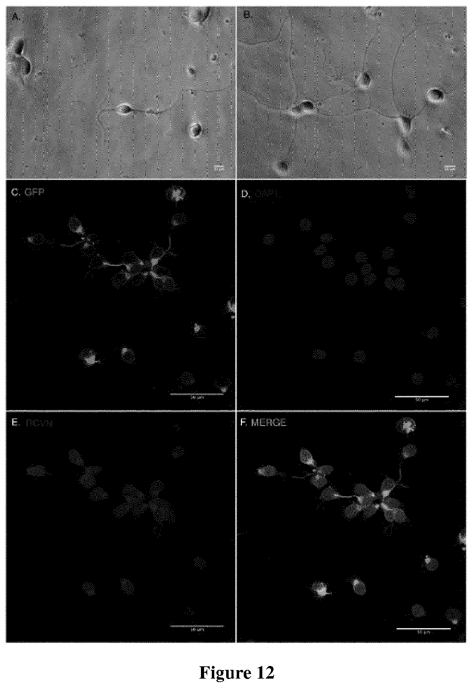

**Figure 12** **:** Generation of Jaws positive photoreceptor monolayers (A-B) Bright field images of D100 monolayer cultures obtained from dissociation of day 70 retinal organoids (C) Immunofluorescence of D100 monolayer cultures obtained from dissociation of day 70 retinal organoids stained against GFP (C), DAPI (D), photoreceptor marker RECOVERIN (E) and merge of the 3 channels (F). Scale bars, A-B 10 um, C-F 50 um

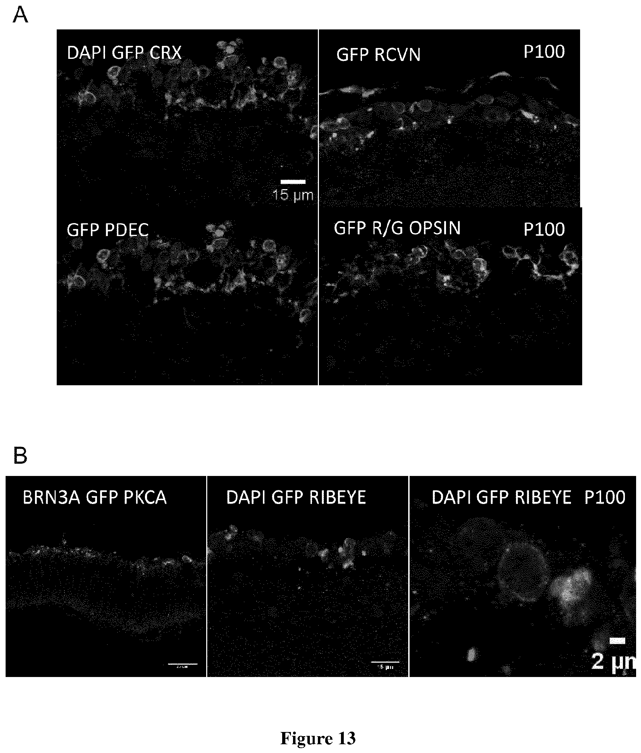

**Figure 13:** (A) Immunostainings on retinal sections from 100 days old rd10 mice transplanted with infected retinal organoid labelling Jaws (GFP) and CRX; Jaws and RCVN, Jaws and PDE6C and Jaws and R/G OPSIN. (B) Immunostainings on retinal sections from 100 days old transplanted rd10 mice labeling Jaws (GFP) and PKCA and Jaws and RIBEYE.

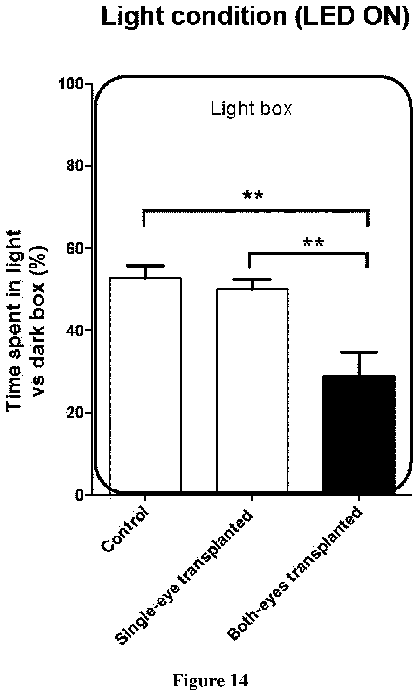

**Figure 14****:** Light/Dark box behavior test on 90 days old rd10 mice. (A). Time spent in light box (%) under the light condition by non-transplanted rd10 mice (n=12), single-eye transplanted rd10 mice (n=10), rd10 mice transplanted in both eyes (n=3) (B). ^{∗}: p < 0,0070 and ^{∗∗}: p < 0,0091

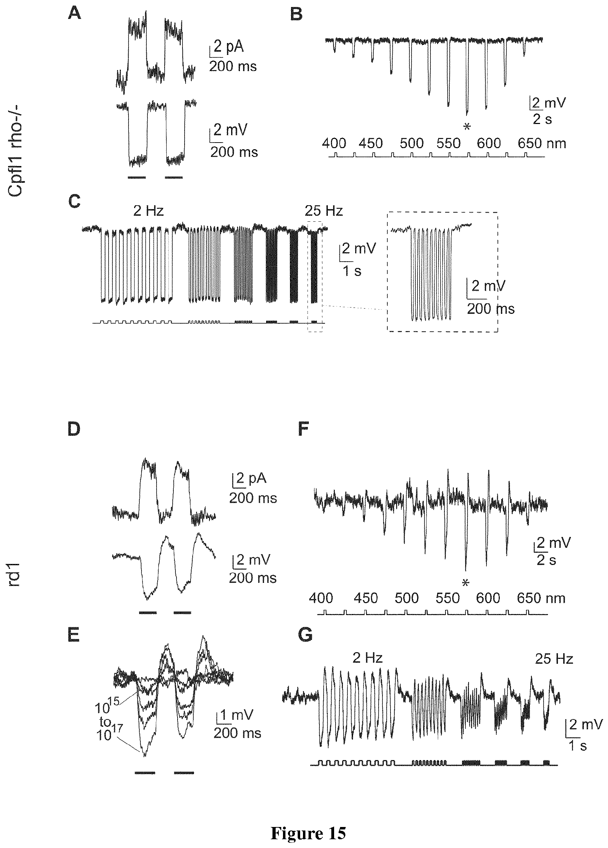

**Figure 15****:** Integrated transplanted Jaws positive photoreceptors derived from hiPSCs respond to light. (A-D) *Cpfl1*/*Rho*^{*-*/*-*} model, (E-G) *rd1* model. (A, D) Representative photocurrents (top) and voltage hyperpolarization (bottom) from cells expressing Jaws stimulated with 2 consecutive flashes of 590 nm light at an intensity of 3.5 × 10¹⁷ photons cm⁻² s⁻¹. (B, F) Jaws-induced hyperpolarization action spectrum corresponding to a Jaws-expressing cell stimulated at different wavelengths. Stimuli ranging from 400 nm to 650 nm, separated by 25 nm steps, were used at 3.5 × 10¹⁷ photons cm⁻² s⁻¹. Maximal responses were obtained at 575 nm. (C, G) Temporal properties: Modulation of Jaws-induced hyperpolarization at increasing stimulation frequencies in transplanted blind mouse (from 2 to 30 Hz; at 3.5 × 10¹⁷ photons cm⁻² s⁻¹), a magnified trace is shown for 25 Hz in (C). (E) Jaws voltage-responses as a function of light stimulation intensity (from 10¹⁴ to 10¹⁷ photons cm⁻² s⁻¹).

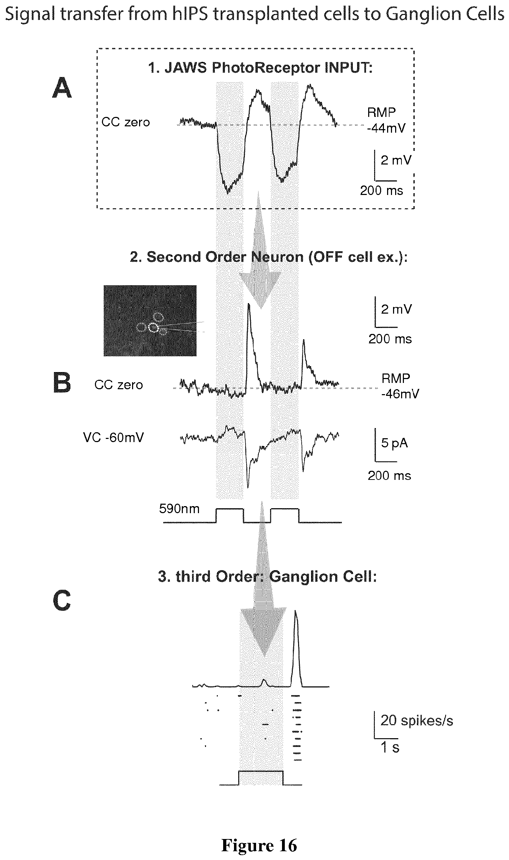

**Figure 16****:** Schematic view of the signal transfer from hiPS to ganglion cells through interneurons. (A-C) Successive electrophysiological recordings in rd1 mouse transplanted with hiPS cells, from 3 different cell types responding to 590 nm light stimuli at 10¹⁷ photons cm⁻² s⁻¹, showing in this example how the INPUT signal from transplanted cells could be transmitted to OFF INL cells and finally to OFF ganglion cells. (A) Representative photocurrents from a cell expressing Jaws stimulated with 2 consecutive flashes. (B) Representative currents (bottom) and voltage (top)responses from a second order cell (OFF cell) in the INL layer that was not expressing Jaws but was very likely connected to surroundings Jaws-hiPS transplanted cells (left image). (C) Example of an OFF-ganglion cell (third order neuron) response (spiking activity) recording that could hypothetically receive its input from the second order neuron displayed in (B).

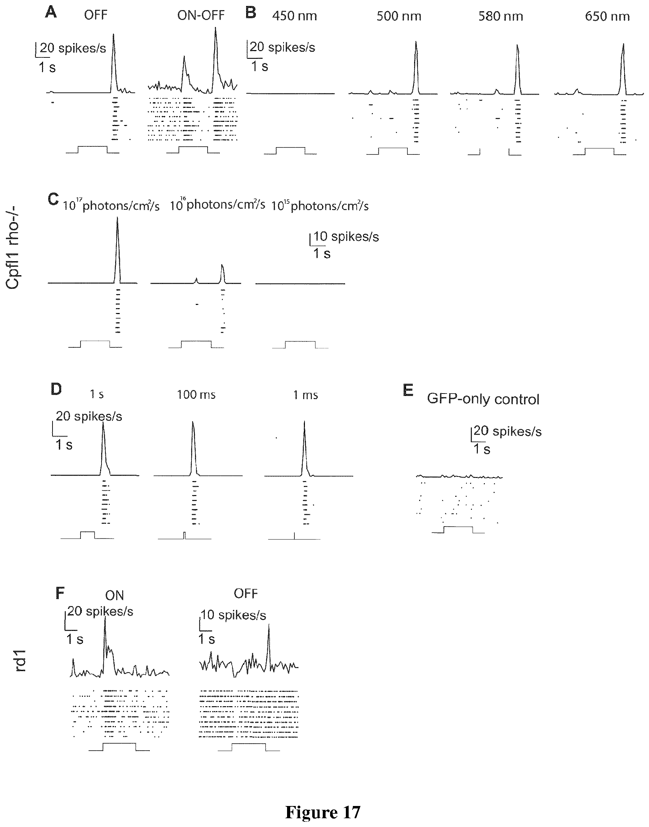

**Figure 17****:** Light responses mediated by Jaws are transmitted to retinal ganglion cells. (A-D) Averaged spike responses obtained from MEA recordings shown as PSTH and raster plots from a transplanted blind Cpfl1/Rho^{-/-} mouse. (A) Representative examples of two RGCs responding either with an OFF-response (left) or an ON/OFF-response (right) in *Cpfl1*/*Rho*^{*-*/*-*} mouse (580 nm; 1.24× 10¹⁷ photons cm⁻² s⁻¹). (B) Representative PSTH and raster plots constructed for wavelengths ranging from 450 to 650 nm (1.24 ×10¹⁷ photons cm⁻² s⁻¹). (C) Averaged spike responses and raster plots for a representative cell at lower light intensities (580 nm; 10¹⁶ photons cm⁻² s⁻¹ and 10¹⁵ photons cm⁻² s⁻¹, respectively) and (D) and shorter light pulses ranging from 1s to 1ms (580 nm; 1.24 × 10¹⁷ photons cm⁻² s⁻¹). (E) Representative PSTH and raster plots recorded from an unresponsive cell from a control retina transplanted with GFP-only-expressing iPSC-derived photoreceptors. (F) Averaged spike responses obtained from multi-electrode array (MEA) recordings shown as PSTH (peristimulus time histogram) and raster plots recorded in transplanted rd1 mice (580 nm; 1.24 × 10¹⁷ photons cm⁻²s⁻¹) - representative traces from two RGCs responding either with an ON-response (left), or an OFF-response (right).

### Detailed description of the invention

The inventors herein demonstrated that the treatment of retinal degenerative diseases can be greatly improved using a new therapeutic strategy consisting to combine cell replacement strategy with the introduction of an optogenetic inhibitor in photoreceptor precursor cells before transplantation. This approach improves the benefices of transplantation of these cells on photoreceptor hyperpolarization.

The inventors showed that transplantation of photoreceptor precursors expressing a microbial opsin in mouse models of severe retinal degeneration enables to restore visually guided behavior in these treated mice. They also observed that these transplanted photoreceptors can integrate well into the retina of the host animal, can form synaptic connections to the remaining inner retinal circuitry and that signals induced by the microbial opsin are transmitted to the output neurons of the blind mice.

### Definitions

As used herein, the term "nucleic acid" or "polynucleotide" refers to a polymeric form of nucleotides of any length, either ribonucleotides or deoxyribonucleotides. Thus, this term includes, but is not limited to, single-, double- or multi- stranded DNA or RNA, genomic DNA, cDNA, DNA-RNA hybrids, or a polymer comprising purine and pyrimidine bases, or other natural, chemically or biochemically modified, non-natural, or derived nucleotide bases. The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidates and thus can be an oligodeoxynucleoside phosphoramidate (P-NH2) or a mixed phosphoramidatephosphodiester oligomer. The nucleic acid of the invention can be prepared by any method known to one skilled in the art, including chemical synthesis, recombination, and mutagenesis. In preferred embodiments, the nucleic acid of the invention is a DNA molecule, preferably a double stranded DNA molecule, and preferably synthesized by recombinant methods well known to those skilled in the art.

As used herein, the term "promoter" refers to a regulatory element that directs the transcription of a nucleic acid to which it is operably linked. A promoter can regulate both rate and efficiency of transcription of an operably linked nucleic acid. A promoter may also be operably linked to other regulatory elements which enhance ("enhancers") or repress ("repressors") promoter-dependent transcription of a nucleic acid. As used herein, the term "specific promoter" shall be understood to refer to a promoter mainly active in a given tissue or cell type. It shall be understood that a residual expression, generally lower, in other tissues or cells cannot be entirely excluded. As used herein, the term "specific photoreceptor promoter" shall be understood to refer to a promoter having a transcriptional promoter activity specific of photoreceptor cells or precursors thereof.

The term "subject" or "patient" refers to an animal having retina, preferably to a mammal, even more preferably to a human, including adult, child and human at the prenatal stage.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease. In other embodiments, this term refers to minimizing the spread or worsening of the disease resulting from the administration of one or more therapeutic agents to a subject with such a disease.

In particular, the term "treatment of retinal degenerative disease" may refer to a preservation or an improvement of the light-detecting capacity of the photoreceptors. In particular, this term may refer to a restoration, improvement or preservation of vision.

By a "therapeutically efficient amount" is intended an amount of a therapeutic agent, e.g. photoreceptor precursor cells of the invention, administered to a subject that is sufficient to constitute a treatment as defined above, in particular a treatment of retinal degenerative disease. In the method of the invention for treating retinal degenerative diseases, the pharmaceutical composition or photoreceptor precursor cells of the invention are preferably administered intraocularly, more preferably by injection in the subretinal space of the eye. In particular photoreceptor precursor cells or pharmaceutical composition are preferably injected between the neural retina and the overlying RPE.

In a first aspect, the present invention relates to an isolated photoreceptor precursor cell, comprising a heterologous nucleic acid encoding an optogenetic inhibitor as defined in the claims.

As used herein, the term "isolated", in relation to a photoreceptor precursor cell, refers to a photoreceptor precursor cell which is not in its natural environment, i.e. which is not in a subject or patient. An isolated photoreceptor precursor cell may be, for example, in a cell culture, in a cell suspension or in a pharmaceutical composition. An isolated photoreceptor precursor cell may have interactions with other cells or cell types. In particular, it may be comprised in an *ex vivo* or *in vitro* cell system, such as an organoid or tissue.

The photoreceptor precursor cells are not-fully differentiated, non-dividing cells committed to differentiate into photoreceptor cells. As used herein, the term "photoreceptor cell" refers to a cone photoreceptor cell or a rod photoreceptor cell. Thus, as used herein, the term "photoreceptor precursor cell" may refer to a rod photoreceptor precursor cell or to a cone photoreceptor precursor cell. The photoreceptor precursor cells are preferably post-mitotic photoreceptor precursors, i.e. cells that are specified to differentiate into rod photoreceptors. Preferably, photoreceptor precursor cells express CD73. More preferably, photoreceptor precursor cells express CD73 and CD24.

In an embodiment, photoreceptor precursor cells were obtained from retina of donor (e.g. cadaver eye donor) or subject to be treated, preferably from the subject to be treated.

In another embodiment, photoreceptor precursor cells were obtained from stem cells, in particular embryonic stem cells, induced pluripotent stem (iPS cells), adult stem cells or fetal stem cells. In another embodiment, photoreceptor precursor cells were obtained from differentiated embryonic stem cells.

Producing photoreceptor precursor cells from human embryonic stem cells may meet ethical challenges. According to one embodiment, embryonic stem cells are non-human embryonic stem cells. According to another embodiment, human embryonic stem cells may be used with the proviso that the method itself or any related acts do not include destruction of human embryos.

Embryonic stem cells can be derived from the inner cell mass of the pre-implantation blastocyst. Embryonic stem cells are able to maintain an undifferentiated state or can be directed to mature along lineages deriving from all three germ layers, ectoderm, endoderm and mesoderm. In the present invention, embryonic stem cells can be directed towards photoreceptor by manipulation of key developmental signaling pathways, for example by using antagonists of the nodal and wnt pathway in addition to activin-A and serum (Watanabe K et al. Nat Neurosci. 2005 Mar;8(3):288-96.), by inhibition of the Notch signaling pathway (Osakada F et al. Nat Protoc. 200;4(6):811-24). Photoreceptor precursor cells could be obtained from embryonic stem cells using any protocol known by the skilled person (Osakada F et al. Nat Biotechnol. 2008 Feb;26(2):215-24; Amirpour N et al. Stem Cells Dev. 2012 Jan;21(1):42-53; Nakano T et al. Cell Stem Cell. 2012 Jun 14;10(6):771-85; Zhu Y et al. Plos One. 2013;8(1):e54552; Yanai A et al. Tissue Eng Part C Methods. 2013 Oct;19(10):755-64; Kuwahara A et al. Nat Commun. 2015 Feb 19;6:6286; Mellough CB et al. Stem Cells. 2015 Aug;33(8):2416-30; Singh RK et al. Stem Cells Dev. 2015 Dec 1;24(23):2778-95).

Preferably, photoreceptor precursor cells were obtained from iPS cells or adult stem cells, more preferably from iPS cells.

Induced pluripotent stem (iPS) cells are derived from a non-pluripotent cell, typically an adult somatic cell, by a process known as reprogramming, where the introduction of only a few specific genes are necessary to render the cells pluripotent (e.g. OCT4, SOX2, KLF4 and C-MYC in human cells). One benefit of use of iPS cells is the avoidance of the use of embryonic cells altogether and hence any ethical questions thereof.

Therefore, according to a preferred embodiment, photoreceptor precursor cells are iPS cell derived photoreceptor precursor cells. iPS cells may have been obtained from the subject to be treated or from another subject. Preferably, iPS cells were derived from cells from the subject to be treated, in particular from fibroblasts of this subject.

Photoreceptor precursor cells could be obtained from iPS cells using any differentiation method known by the skilled person. In particular, photoreceptor precursor cells could be obtained from human iPS cells which are expanded to confluence in iPS medium (e.g. Essential 8^{™} medium, GIBCO, Life Technologies). After confluence, the medium was switched to a proneural medium (e.g. Essential 6^{™} medium supplemented with 1 % N2 supplement; GIBCO, Life Technologies) for 28 days. On D28, identified neural-retinal like structures were isolated using a needle and were plated in maturation medium (e.g. DMEM/Nutrient Mixture F-12, 1% MEM nonessential amino acids, 2% B27 supplement; (Life Technologies) supplemented with FGF2. At day 35, FGF2 was removed and retinal organoids were further cultured in maturation medium with the gamma-secretase inhibitor N-[N-(3,5-difluorophenacetyl)-l-alanyl]-S-phenylglycine t-butyl ester (DAPT) which was added from day 42 to day 49 of differentiation. Transplantable photoreceptor precursors of human origin are obtained at day 70.

Photoreceptor precursor cells could also be obtained from human iPS cells using any other protocol known by the skilled person (Lamba, Osakada and colleagues (Lamba et al. Proc Natl Acad Sci USA. 2006 Aug 22;103(34):12769-74;, Lamba et al. Plos one. 2010 Jan 20;5(1):e8763; Osakada et al. Nat. Protoc. 2009;4(6):811-24; Meyer JS et al. Proc Natl Acad Sci USA. 2009 Sep 29; 106(39):16698-703 ; Meyer JS et al. Stem Cells. 2011 Aug ;29(8) :1206-18; Mellough CB et al. Stem Cells. 2012 Apr;30(4):673-86; Boucherie C et al. Stem Cells. 2013 Feb ;31(2) :408-14; Sridhar A et al. Stem Cells Transl Med. 2013 ;2(4) :255-64 ; Tucker BA et al. Elife. 2013 Aug 27,2:e00824 ; Tucker BA et al. Stem Cells Transl Med. 2013 Jan ;2(1) :16-24; Reichman S et al. Proc Natl Acad Sci USA. 2014 Jun 10 ;111(23) :8518-23 ; Zhong X et al. Nat Commin. 2014 Jun 10 ;5 :4047 ; Wang X et al. Biomaterials. 2015 Jun ;53 :40-9).

Photoreceptor precursor cells of the present invention comprise a heterologous nucleic acid encoding an optogenetic inhibitor as defined in the claims.

As used herein, the term « heterologous nucleic acid » refers to a gene, polynucleotide or nucleic acid sequence that is not in its natural environment. In particular, this term refers to a nucleic acid molecule which is not naturally present in photoreceptor cells or photoreceptor precursors. In other words, it refers to a nucleic acid, which originates from a foreign source or species or, if from the same source, is modified from its original form.

The heterologous nucleic acid may be any nucleic acid sequence encoding an optogenetic inhibitor as defined in the claims.

As used herein, the term "optogenetic tool" refers to a molecule that changes the membrane potential of target cells following an increase in light intensity. The molecule may hyperpolarize or depolarize the target cell. As used herein, the term "optogenetic inhibitor" refers to an optogenetic tool that hyperpolarizes neurons in which it is expressed, and thus inactivates them.

An optogenetic inhibitor causes a cell to hyperpolarize upon exposure to light. When a cell hyperpolarizes, the negative internal charge of the cell becomes more negative for a brief period. The shift to more negative inhibits action potentials by increasing the stimulus required to move the membrane potential to the action potential threshold. In a particular embodiment, an optogenetic inhibitor is a light-gated ion pump that upon absorption of a photon transports chloride ions inward and/or transports cations outward. A light-gated, retinal-dependent, ion pump that transports chloride ions inward or cations outward upon absorption of a photon may thus be an optogenetic inhibitor.

In the context of the present invention, the optogenetic inhibitor is selected from the group consisting of halorhodopsins such as halorhodopsin from the archea *Natromonas pharaonis* (NpHR), enhanced halorhodopsins (eNpHR2.0 and eNpHR3.0) and the redshifted halorhodopsin Halo57, archaerhodopsin-3 (AR-3), archaerhodopsin (Arch), bacteriorhodopsins such as enhanced bacteriorhodopsin (eBR), proteorhodopsins, xanthorhodopsins, Leptosphaeria maculans fungal opsins (Mac), the cruxhalorhodopsin Jaws also named Jaws. Preferably, the optogenetic inhibitor is selected from halorhodopsins such as enhanced halorhodopsins (eNpHR2.0 and eNpHR3.0) and the redshifted halorhodopsin Halo57, and Jaws, more preferably from enhanced halorhodopsins and Jaws.

Some of the optogenetic inhibitors described herein are natural proteins without modifications.

In a particular embodiment, photoreceptor precursor cells are genetically engineered by introducing an expression cassette or expression vector comprising the heterologous nucleic acid sequence encoding optogenetic inhibitor into said cells. As used herein, the term "expression cassette" refers to a nucleic acid construct comprising a coding sequence and one or more control sequences required for expression of said coding sequence. In particular, one of these control sequence is a promoter driving the expression of the coding sequence.

Preferably, the nucleic acid sequence encoding optogenetic inhibitor is operably linked to a promoter that is recognized by the photoreceptor precursor cells. The promoter contains transcriptional control sequences that mediate the expression of the optogenetic inhibitor. The promoter may be any polynucleotide that shows transcriptional activity in photoreceptor precursor cells including mutant, truncated, and hybrid promoters. The promoter may be a constitutive or inducible promoter, preferably a constitutive promoter.

Examples of suitable promoters include, but are not limited to, rhodopsin promoter, cone arrestin promoter, cGMP phosphodiesterase (PDE) promoter, interphotoreceptor retinoid-binding protein (IRBP) promoter, the SV40 promoter, the CMV promoter, the dihydrofolate reductase promoter, the phosphoglycerol kinase promoter, the RPE-65 promoter, the tissue inhibitor of metalloproteinase 3 (Timp3) promoter and the tyrosinase promoter.

In a particular embodiment, the promoter is tissue-specific, in particular specific of photoreceptor cells or photoreceptor precursor cells. Preferably, said specific promoter is selected from the group consisting of rhodopsin promoter, cone arrestin promoter, cGMP phosphodiesterase (PDE) promoter, interphotoreceptor retinoid-binding protein (IRBP) promoter.

In a preferred embodiment, the promoter is selected from the group consisting of rhodopsin promoter and cone arrestin promoter. In a particular embodiment, photoreceptor precursor cells are rod photoreceptor precursor cells and the promoter is rhodopsin promoter. In another particular embodiment, photoreceptor precursor cells are cone photoreceptor precursor cells and the promoter is cone arrestin promoter.

The expression cassette may also include appropriate transcription initiation, termination, and enhancer sequences; efficient RNA processing signals such as splicing and polyadenylation signals; sequences that stabilize cytoplasmic mRNA; sequences that enhance translation efficiency (i.e., Kozak consensus sequence); and/or sequences that enhance protein stability. A great number of expression control sequences, e.g., native, constitutive, inducible and/or tissue- specific, are known in the art and may be utilized to drive expression of the nucleic acid sequence encoding optogenetic inhibitor.

The nucleic acid sequence or expression cassette may be contained in an expression vector. As used herein, the term "vector" refers to a nucleic acid molecule used as a vehicle to transfer genetic material, and in particular to deliver a nucleic acid into a host cell, either in vitro or in vivo. The vector may be an autonomously replicating vector, i.e., a vector that exists as an extra-chromosomal entity, the replication of which is independent of chromosomal replication, e.g., a plasmid, an extra-chromosomal element, a mini-chromosome, or an artificial chromosome. The vector may contain any means for assuring self-replication. Alternatively, the vector may be one that, when introduced into the host cell, is integrated into the genome and replicated together with the chromosome(s) into which it has been integrated.

Preferably, the heterologous nucleic acid, expression cassette or vector is integrated into the genome of the host cell and replicated together with the chromosome(s) into which it has been integrated. Examples of appropriate vectors include, but are not limited to, recombinant integrating or non-integrating viral vectors and vectors derived from recombinant bacteriophage DNA, plasmid DNA or cosmid DNA. Preferably, the vector is a recombinant integrating or non-integrating viral vector. Examples of recombinant viral vectors include, but not limited to, vectors derived from herpes virus, retroviruses, lentivirus, vaccinia viruses, adenoviruses, adeno-associated viruses or bovine papilloma virus.

Preferably, the heterologous nucleic acid encoding optogenetic inhibitor or the expression cassette is contained in a recombinant adenovirus, adeno-associated virus or lentivirus vector.

In a preferred embodiment, the vector is a recombinant adeno-associated virus (AAV) vector, i.e. the photoreceptor precursor cells of the invention are AAV transduced photoreceptor precursor cells expressing an optogenetic inhibitor.

The human parvovirus Adeno-Associated Virus (AAV) is a dependovirus that is naturally defective for replication which is able to integrate into the genome of the infected cell to establish a latent infection. The last property appears to be unique among mammalian viruses because the integration occurs at a specific site in the human genome, called AAVSI, located on chromosome 19 (19ql3.3-qter). Therefore AAV has arisen considerable interest as a potential vector for human gene therapy. Among the favorable properties of the virus are its lack of association with any human disease, its ability to infect both dividing and non-dividing cells, and the wide range of cell lines derived from different tissues that can be infected.

As used herein, the term "AAV vector" refers to a polynucleotide vector comprising one or more heterologous sequences (i.e., nucleic acid sequence not of AAV origin, e.g. the sequence encoding the optogenetic inhibitor) that are flanked by at least one AAV inverted terminal repeat sequence (ITR), preferably two ITRs. Such AAV vectors can be replicated and packaged into infectious viral particles when present in a host cell that has been infected with a suitable helper virus (or that is expressing suitable helper functions) and that is expressing AAV rep and cap gene products (i.e. AAV Rep and Cap proteins). An "inverted terminal repeat" or "ITR" sequence is a term well understood in the art and refers to relatively short sequences found at the termini of viral genomes which are in opposite orientation. An "AAV inverted terminal repeat (ITR)" sequence is an approximately 145-nucleotide sequence that is present at both termini of the native single-stranded AAV genome. The outermost 125 nucleotides of the ITR can be present in either of two alternative orientations, leading to heterogeneity between different AAV genomes and between the two ends of a single AAV genome. The outermost 125 nucleotides also contains several shorter regions of self-complementarity (designated A, A', B, B', C, C and D regions), allowing intra-strand base-pairing to occur within this portion of the ITR. AAV ITRs may have a wild-type nucleotide sequence or may be altered by the insertion, deletion or substitution. The serotype of the inverted terminal repeats (ITRs) of the AAV vector may be selected from any known human or nonhuman AAV serotype.

The vector may further comprise one or more nucleic acid sequences encoding selectable marker such as auxotrophic markers (e.g., LEU2, URA3, TRP 1 or HIS3), detectable labels such as fluorescent or luminescent proteins (e.g., GFP, eGFP, DsRed, CFP, YFP), or protein conferring resistance to a chemical/toxic compound (e.g., MGMT gene conferring resistance to temozolomide). These markers can be used to select or detect host cells comprising the vector and can be easily chosen by the skilled person according to the host cell.

The vector may be packaged into a virus capsid to generate a "viral particle". In particular, the vector may be an AAV vector packaged into an AAV-derived capsid to generate an "adeno-associated viral particle" or "AAV particle" composed of at least one AAV capsid protein and an encapsidated AAV vector genome.

The capsid serotype determines the tropism range of the AAV particle. Multiple serotypes of adeno-associated virus (AAV), including 12 human serotypes and more than 100 serotypes from nonhuman primates have now been identified (Howarth al., 2010, Cell Biol Toxicol 26: 1-10). Among these serotypes, human serotype 2 was the first AAV developed as a gene transfer vector. Other currently used AAV serotypes include, but are not limited to, AAV1, AAV3, AAV4, AAV5, AAV6, AAV7, AAV8, AAVrh8, AAV9, AAV10, AAVrh10, AAV11, AAV12, AAVrh74 and AAVdj, etc..

In a particular embodiment, the AAV vector comprises an AAV-derived capsid selected from the group consisting of AAV2, AAV9, AAV9-2YF, AAV5, AAV2-7m8 (Dalkara D et al. Gene Ther. 2012 Feb;19(2):176-81; Dalkara D et al. Sci Transl Mes. 2013 Jun 12;5(189):189ra76) or AAV8 capsid.

In addition, non-natural engineered variants and chimeric AAV can also be useful. In particular, the capsid proteins may be variants comprising one or more amino acid substitutions to enhance transduction efficiency, to minimize immunogenicity, to tune stability and particle lifetime, for efficient degradation and/or for accurate delivery to the nucleus. Mutated AAV capsids may be obtained from capsid modifications inserted by error prone PCR and/or peptide insertion or by including one or several amino acids substitutions. In particular, mutations may be made in any one or more of tyrosine residues of natural or non-natural capsid proteins (e.g. VP1, VP2, or VP3). Preferably, mutated residues are surface exposed tyrosine residues. Exemplary mutations include, but are not limited to tyrosine-to-phenylalanine substitutions such as Y252F, Y272F, Y444F, Y500F, Y700F, Y704F, Y730F, Y275F, Y281F, Y508F, Y576F, Y612G, Y673F and Y720F.

Alternatively to using AAV natural serotypes, artificial AAV serotypes may also be used including, without limitation, AAV with a non-naturally occurring capsid protein. Such an artificial capsid may be generated by any suitable technique, using a selected AAV sequence (e.g., a fragment of a VPI capsid protein) in combination with heterologous sequences which may be obtained from a different selected AAV serotype, non-contiguous portions of the same AAV serotype, from a non-AAV viral source, or from a non-viral source. An artificial AAV serotype may be, without limitation, a chimeric AAV capsid or a mutated AAV capsid. A chimeric capsid comprises VP capsid proteins derived from at least two different AAV serotypes or comprises at least one chimeric VP protein combining VP protein regions or domains derived from at least two AAV serotypes. An AAV particle can comprise viral proteins and viral nucleic acids of the same serotype or a mixed serotype (i.e. pseudotyped AAV). For example, the recombinant AAV vector may be an AAV serotype 2/1 hybrid recombinant gene delivery system comprising AAV2 genome and AAV1 capsid proteins. Those skilled in the art are familiar with such vectors and methods for their construction and use, see e.g. WO 01/83692.

The AAV vector for use in the present invention may be easily chosen by the skilled person.

In a preferred embodiment, the AAV vector comprising the heterologous nucleic acid encoding the optogenetic inhibitor or the expression cassette, is an AAV vector of serotype 2 or 9, or an AAV vector derived from AAV-2 or AAV-9, such as, for example, AAV9-2YF or AAV2-7m8 (Dalkara D et al. Gene Ther. 2012 Feb;19(2):176-81; Dalkara D et al. Sci Transl Mes. 2013 Jun 12;5(189):189ra76).

The nucleic acid construct, expression cassette or vector may be transferred into photoreceptor precursor cells using any known technique including, but being not limited to, calcium phosphate -DNA precipitation, DEAE-Dextran transfection, electroporation, microinjection, biolistic, lipofection, or viral infection.

In a further aspect, the present invention also provides a pharmaceutical composition comprising photoreceptor precursor cells of the present invention and a pharmaceutically acceptable excipient.

All the embodiments described above are also contemplated in this aspect. The pharmaceutically acceptable excipient is selected according to the route of administration. As used herein, the term "pharmaceutically acceptable" means approved by a regulatory agency or recognized pharmacopeia such as European Pharmacopeia, for use in animals and/or humans. The term "excipient" refers to a diluent, adjuvant, carrier, or vehicle with which the therapeutic agent is administered. As is well known in the art, pharmaceutically acceptable excipients are relatively inert substances that facilitate administration of a pharmacologically effective substance and can be supplied as liquid solutions or suspensions, as emulsions, or as solid forms suitable for dissolution or suspension in liquid prior to use. For example, an excipient can give form or consistency, or act as a diluent. Suitable excipients include but are not limited to stabilizing agents, wetting and emulsifying agents, salts for varying osmolality, encapsulating agents, pH buffering substances, and buffers. Such excipients include any pharmaceutical agent suitable for direct delivery to the eye which may be administered without undue toxicity.

Preferably, the composition is formulated to be administered by intraocular injection, in particular to the subretinal space of the eye, preferably between the retina and the overlying RPE. Pharmaceutical composition suitable for such administration may comprise the photoreceptor precursor cells, in combination with one or more pharmaceutically acceptable sterile isotonic aqueous (e.g. isotonic 0.9% NaCl) or nonaqueous solutions (e.g., balanced salt solution (BSS)), dispersions, suspensions or emulsions, or sterile powders which may be reconstituted into sterile injectable solutions or dispersions just prior to use, which may contain antioxidants, buffers, bacteriostats, solutes or suspending or thickening agents. A thorough discussion of pharmaceutically acceptable excipients is available in Remington's Pharmaceutical Sciences, 15th Edition.

Optionally, the pharmaceutical composition comprising photoreceptor precursor cells of the invention may be frozen for storage at any temperature appropriate for storage of the cells. For example, the cells may be frozen at about -20°C, -80°C or any other appropriate temperature. Cryogenically frozen cells may be stored in appropriate containers and prepared for storage to reduce rick of cell damage and maximize the likelihood that the cells will survive thawing. Alternatively, the cells may also be maintained at room temperature of refrigerated, e.g. at about 4°C.

The amount of photoreceptor precursor cells to be administered may be determined by standard procedure well known by those of ordinary skill in the art. Physiological data of the patient (e.g. age, size, and weight) and type and severity of the disease being treated have to be taken into account to determine the appropriate dosage.

The pharmaceutical composition of the invention may be administered as a single dose or in multiple doses. In particular, each unit dosage may contain, from 100,000 to 300,000 photoreceptor precursor cells per µl, preferably from 200,000 to 300,000 photoreceptor precursor cells per µl.

The pharmaceutical composition may further comprise one or several additional active compounds such as corticosteroids, antibiotics, analgesics, immunosuppressants, trophic factors, or any combinations thereof.

In another aspect, the present invention also relates to an *in vitro* method of producing photoreceptor precursor cells of the invention, said method comprising introducing a nucleic acid encoding optogenetic inhibitor, or an expressing cassette or vector comprising said nucleic acid, into provided photoreceptor precursor cells, as defined in the claims. In an embodiment, photoreceptor precursor cells to be modified by introducing said nucleic acid were obtained from a retina, in particular from a donor or a patient to be treated.

In another embodiment, photoreceptor precursor cells to be modified were obtained from differentiation of stem cells, preferably from differentiation of adult stem cells or induced pluripotent stem cells, more preferably from differentiation of induced pluripotent stem cells obtained from somatic cells, e.g. fibroblasts, of the subject to be treated.

Said photoreceptor precursor cells may have been purified or isolated by any method known by the skilled person, e.g. by isolating CD73 positive cells from retinal cells or retinal organoid, in particular by using anti-CD73 antibodies.

In a particular embodiment, the method of producing photoreceptor precursor cells of the invention comprises introducing into provided photoreceptor precursor cells a heterologous nucleic acid encoding optogenetic inhibitor, or an expression cassette or vector comprising said nucleic acid, as described above, wherein the photoreceptor precursor cells were obtained from differentiation of stem cells, preferably from differentiation of adult stem cells or induced pluripotent stem cells obtained from somatic cells, e.g. fibroblasts, from the patient to be treated.

The method may further comprise preparing a pharmaceutical composition according to the invention by adding a pharmaceutically acceptable excipient as described above to said genetically modified photoreceptor precursor cells.

The method may comprise differentiating induced pluripotent stem cells, that were provided from the patient to be treated, into photoreceptor precursor cells. The induced pluripotent stem cells may have been obtained from adult somatic cells, preferably fibroblasts, from the patient to be treated.

In a particular embodiment, the heterologous nucleic acid encoding optogenetic inhibitor or the expression cassette is comprised in a recombinant adeno-associated virus (AAV) vector.

The nucleic acid construct, expression cassette or vector may be transferred into photoreceptor precursors cells using any known technique including, but being not limited to, calcium phosphate - DNA precipitation, DEAE-Dextran transfection, electroporation, microinjection, biolistic, lipofection, or viral infection.

All the embodiments of the photoreceptor precursor cells and the pharmaceutical composition are also contemplated in this method.

In another aspect, the present invention further relates to photoreceptor precursor cells of the invention or a pharmaceutical composition of the invention for use in the treatment of retinal degenerative disease.

All the embodiments described above for the photoreceptor precursor cells of the invention, the pharmaceutical composition of the invention and the method of producing photoreceptor precursor cells of the invention are also contemplated in this aspect.

The retinal degenerative disease is preferably related to a loss of function or death of photoreceptors, and more preferably is related to a loss of function of photoreceptors.

Retinal degenerative diseases include, but are not limited to, macular degeneration, retinitis pigmentosa (syndromic and non-syndromic), cone dystrophy, Usher syndrome, rod dystrophy, rod-cone dystrophy, achromatopsia and Bardet-Biedl syndrome. Preferably, the retinal degenerative disease is retinitis pigmentosa or age related macular degeneration.

In an embodiment, photoreceptor precursors or pharmaceutical composition as described above are particularly suitable to be used for the treatment of a retinal degenerative disease in patient with advanced stages of photoreceptor degeneration. Advanced stage of photoreceptor degeneration may be characterized mainly by a substantial loss of photoreceptor cells but also by the presence of retinal glial scar and significant changes in the outer layer membrane. Patients with advanced stages of photoreceptor degeneration may exhibit a dramatic impairment of vision or even a complete loss of vision.

In a particular embodiment, photoreceptor precursor cells of the invention used in the treatment of a retinal degenerative disease were obtained from cells of the patient to be treated. Photoreceptor precursor cells may have been obtained from the patient as described above, from a sample of retina of from stem cells of said patient. Preferably, photoreceptor precursor cells used in the present invention were obtained from stem cells of the patient to be treated, more preferably from adult stem cells or from induced pluripotent stem cells derived from adult somatic cells of the patient such as fibroblasts.

Thus, in a particular embodiment, (i) photoreceptor precursor cells were obtained from differentiation of stem cells, preferably from differentiation of adult stem cells or induced pluripotent stem cells obtained from somatic cells, e.g. fibroblasts, from the patient to be treated and (ii) a heterologous nucleic acid encoding optogenetic inhibitor, or an expression cassette or vector comprising said nucleic acid, as described above, was introduced into said cells.

Induced pluripotent stem cells from the patient to be treated may have been differentiated into photoreceptor precursor cells.

Adult somatic cells, preferably fibroblasts, from the patient to be treated may have been provided in order to obtain induced pluripotent stem cells. Adult somatic cells, preferably fibroblasts, obtained from the patient may be reprogrammed into pluripotent stem cells by any method known by the skilled person, preferably by expressing into said cells specific genes (e.g. OCT4, SOX2, C-MYC and KLF4).

In a particular embodiment said retinal degenerative disease is inherited retinal degenerative disease such as inherited retinitis pigmentosa or inherited age-related macular degeneration. In particular said inherited retinal degenerative disease may be due to a mutation within genes which include, but are not limited to, *ABCA4, EYS, PDE6B, RPE65, RHO, USH2A, RPGR, WFS1, CRB1.* In this particular embodiment, photoreceptor precursor cells may have been obtained from the inherited retinal degenerative disease patient to be treated and may have been genetically engineered to correct disease-causing mutation. Said photoreceptor precursor cells can be genetically engineered by any method known by the skilled person, preferably by using homologous recombination of a nucleic acid comprising corrected gene. The genetic correction may have been obtained by introducing into cells a nucleic acid comprising at least a sequence encoding corrected gene and a portion of an endogenous gene such that homologous recombination occurs between the endogenous gene and the nucleic acid. In a particular embodiment, a rare-cutting endonuclease, which is able to cleave a target sequence within an endogenous gene, may have been expressed in the cell. Said rare- cutting endonuclease can be a meganuclease, a TALE-nuclease, a Zinc finger nuclease or a CRISPR/Cas9 endonuclease. In a preferred embodiment, said genetically engineered photoreceptor precursor cells were obtained from induced pluripotent stem cell derived from patient adult somatic cells, preferably fibroblasts. In another embodiment, said genetically engineered photoreceptor precursor cells were obtained from a donor.

In another preferred embodiment, (i) photoreceptor precursor cells were obtained from the patient to be treated as described above, from a sample of retina of from stem cells of said patient, (ii) a heterologous nucleic acid encoding an optogenetic inhibitor, as defined in the claims, was introduced into said cells; and iii) photoreceptor precursor cells were genetically engineered to correct disease-causing mutation. Step (ii) and (iii) may have been conducted sequentially or simultaneously, in any order.

Preferably, photoreceptor precursor cells obtained from the patient to be treated were from differentiation of stem cells, preferably from differentiation of adult stem cells or induced pluripotent stem cells obtained from somatic cells, e.g. fibroblasts, from the patient to be treated.

A therapeutically efficient amount of the photoreceptor precursor cells, genetically engineered to correct disease-causing mutation and comprising a heterologous nucleic acid encoding an optogenetic inhibitor as defined in the claims, may be administered to a subject in need thereof.

In another embodiment, photoreceptor precursor cells comprising a heterologous nucleic acid encoding an optogenetic inhibitor as defined in the claims, used to treat inherited retinal degenerative disease were obtained from a healthy donor, i.e. a donor without mutation causing inherited retinal degenerative disease. In such embodiment, (i) photoreceptor precursor cells were obtained from a healthy donor, i.e. from a sample of retina of from stem cells of said donor, and (ii) a heterologous nucleic acid encoding an optogenetic inhibitor, as defined in the claims, was introduced into said cells.

At least one additional therapeutic agent may be administered to the subject. In particular, said therapeutic agent may be selected from the group consisting of a corticosteroid, an antibiotic, an analgesic, an immunosuppressant, or a trophic factor, or any combinations thereof. Said additional therapeutic agent and photoreceptor precursor cells of the invention may be administered sequentially or simultaneously. When administered simultaneously, the additional therapeutic agent and photoreceptor precursor cells of the invention may be formulated in the same pharmaceutical composition.

Preferably, the photoreceptor precursor cells or the pharmaceutical composition of the invention are administered by intraocular injection, preferably subretinal space injection, more preferably between the neural retina and the overlying RPE.

The amount of photoreceptor precursor cells of the invention to be administered may be determined by standard procedure well known by those of ordinary skill in the art. Physiological data of the patient (e.g. age, size, and weight) and type and severity of the disease being treated have to be taken into account to determine the appropriate dosage.

Engineered photoreceptor precursor cells of the invention may be administered as a single dose or in multiple doses. In particular, each unit dosage may contain, from 100,000 to 300,000 photoreceptor precursor cells per µl, preferably from 200,000 to 300,000 photoreceptor precursor cells per µl.

The following examples are given for purposes of illustration and not by way of limitation.

### Examples:

### A. Optogenetic stimulation triggers halorhodopsin-induced light responses in transplanted photoreceptors of blind CPFL (Cone Photoreceptor Function Loss) mice and rd1 mice.

### 1. Optogenetic transformation of donor (photoreceptor precursor) cells.

At postnatal day P2, C57BL/6J mice (wild type) from Janvier Laboratories (Le Genest Saint Isle, France) were anesthetized on ice, and a cut through the eyelid was made and 1 µl stock containing 3.0 × 10¹³ - 3.3 × 10¹⁴ particles of AAV encoding halorhodopsin under the human rhodopsin promoter (AAV9 2YF-hRho-eNpHR-EYFP) was injected into each eye's vitreous cavity using a ultrafine 34-gauge syringe (Hamilton) (Figure 1, top).

### 2. Donor Cell (photoreceptor precursor) isolation - Enrichment of cells by MACS

Retinas were isolated from the C57BL/6J previously injected mice at postnatal day P4. Retinas were digested using a papain dissociation system (Worthington Biochemical Corporation). The cells (~10⁷ cells/mL) were collected by centrifugation (5 minutes at 300g), resuspended in 500 µL MACS buffer (phosphate-buffered saline [PBS; pH 7.2], 0.5% BSA, 2 mM EDTA) and incubated with 10 µg/mL rat anti-mouse CD73 antibody (BD Biosciences) for 5 minutes at 4°C. After washing in MACS buffer, cells were centrifuged for 5 minutes at 300g. The cell pellet was resuspended in 480 µL MACS buffer and 120 µL goat anti-rat IgG magnetic beads (Miltenyi Biotec). The suspension was incubated for 15 minutes at 4°C followed by a washing step with MACS buffer and centrifugation. Before magnetic separation, the cells were resuspended in MACS buffer and filtered through a 30-µm pre-separation filter. The cell suspensions were applied onto a pre-equilibrated LS column fixed to a MACS separator. The column was rinsed with 3 × 3 mL MACS buffer and the flow through was collected (CD73 negative cells). The column was removed from the magnet and placed in a new collection tube. The CD73-positive fraction was eluted by loading 5 mL MACS buffer and immediately applying the plunger supplied with the column.

### 3. Transplantation into the blind Cpfl1/Rho^{-/}⁻ mice and rd1 mice.

In order to transplant the cell suspension into the subretinal space, adult Cpfl1/rho^{-/-} blind mice or rd1 mice displaying an early onset severe retinal degeneration were anesthetized by an intraperitoneal injection of ketamine (50mg/kg) and xyazine (10mg/kg). Pupils were dilated by drops of tropicamide (Mydriaticum Dispersa), and by using a syringe with a blunt 34-gauge needle (Hamilton), one microliter suspension containing ~200,000 cells was injected into the subretinal space (Figure 1, top).

### 4. Patch-clamp recordings

Isolated retinas were placed in the recording chamber of the microscope at 36°C in oxygenated (95% O₂/5% CO₂) Ames medium (Sigma-Aldrich, St. Louis, MO) during the whole experiment. A custom-made two-photon microscope equipped with a 25x water immersion objective (XLPlanN-25x-W-MP/NA1.05, Olympus, Tokyo, Japan) and a pulsed femto-second laser (InSight^{™} DeepSee^{™} - Newport Corporation, Irvine, CA) was used for patch-clamp recording of YFP-positive cells.

AAV-transduced fluorescent cells were targeted with a patch electrode. Whole-cell recordings were obtained using the Axon Multiclamp 700B amplifier (Molecular Device Cellular Neurosciences, Sunnyvale, CA). Patch electrodes were made from borosilicate glass (BF100-50-10, Sutter Instrument, Novato, CA) pulled to 8-10 MΩ, and filled with 115mM K Gluconate, 10mM KCI, 1mM MgCI2, 0.5mM CaCI2, 1.5mM EGTA, 10mM HEPES, and 4mM ATP-Na2 (pH 7.2). Cells were recorded under voltage-clamp configuration (at -40 mV) to measure photocurrents, or under current-clamp (zero) configuration in order to monitor the membrane potential variation during light stimulations.

### 5. Light stimulation

Subsequent to transplantation, the functionality was tested by a set of electrophysiological experiments.

In order to measure light responses of YFP-positive cells we used a monochromatic light source (Polychrome V, TILL photonics (FEI), Hillsboro, OR). After patching the cells we first stimulated them with a pair of 590 nm full-field light pulses.

Then the activity spectrum was measured by using light flashes ranging from 400 nm to 650 nm (separated by 25 nm steps), a continuous 'rainbow' spectrum between 350 and 680 nm was also used.

Finally we generated light pulses at different frequencies ranging between 2 and 25 Hz to determine the temporal response properties of recorded cells. Stimuli were generated using custom-written software in Matlab (Mathworks, Natick, MA) and Labview (National Instruments, Austin, TX). We used light intensities ranging between 1.3 ×10 ¹⁶ and 3.2 × 10¹⁷ photons cm⁻² s⁻¹.

Figures 2A and 2D show typical light-evoked responses of NpHR-expressing photoreceptor precursors stimulated with two consecutive flash of light at 590 nm integrates in *Cpfl1*/*Rho*^{*-*/*-*} and *rd1* model, respectively, and these cells display photovoltages with spectral tunning with a maximal response obtained at 575 nm (Figures 2B (*Cpfl1*/*Rho*^{*-*/*-*} model); Figure 2E (*rd1* model)). The modulation of Halorhodopsin-induced membrane hyperpolarizations at increasing stimulation frequencies (from 2 to 25Hz; at 1.3×10¹⁶ photons cm⁻²s⁻¹), is shown in Figure 2C *(Cpfl1*/*Rho*^{*-*/*-*} model) and in Figure 2F (*rd1* model).

Figure 2G shows the comparison of light response characteristics between NpHR-photoreceptor precursors transplanted *Cpfl1*/*Rho*^{*-*/*-*} and *rd1* models. Figure 2G left represents mean photocurrent peaks obtained in transplanted cells at -40mV or 0 mV in voltage-clamp configuration and Figure 2G right represents mean peak voltage response obtained in current-clamp configuration (current zero).

Figure 2H shows the comparison of rise and decay time constants between transplanted cells in the two models (in current clamp 'zero' configuration) at 590 nm and 1 × 10¹⁶ photons cm⁻² s⁻¹ (bottom).

Thus, optogenetic stimulation triggers Halorhodopsin-induced light responses in transplanted photoreceptors of blind CPFL mice and rd1 mice, this response is maximal at a light intensity of 575 nm, displays fast kinetics and is not significantly different from the responses obtained from donor's cells.

### 6. Live two-photon-imaging

Isolated retinas or retinal organoids were placed in the recording chamber of the microscope at 36°C in oxygenated (95% O₂; 5% CO₂) Ames medium (Sigma-Aldrich, St. Louis, MO) during the whole experiment. A custom-made two-photon microscope equipped with a 25x water immersion objective (XLPlanN-25x-W-MP/NA1.05, Olympus, Tokyo, Japan) and a pulsed femto-second laser (InSight^{™} DeepSee^{™} - Newport Corporation, Irvine, CA) was used for imaging of YFP-positive cells. Two-photon images were acquired using the excitation laser at a wavelength of 930 nm. Images were processed offline using ImageJ (NIH, Bethesda, MD).

As shown in Figure 3A (with epifluorescence light) and 3B (with two-photon-imaging), Halorhodopsin-transduced progenitor cells survived 20 days after transplantation in the host and fluorescence was restricted to the cell membrane.

### 7. Multi-electrode array recordings and data analysis

All multi-electrode array recordings were performed on a 252 channel multi-electrode array system (USB-MEA256-System, Multi Channel Systems, Reutlingen, Germany) and data was acquired using the MC_Rack software (MC_Rack v4.5, Multi Channel Systems). *Ex vivo* isolated flat mounted retinae of transplanted *Cpfl1*/*Rho*^{*-*/*-*} and *Rd1* mice aged 10 weeks or older were tested. Mice were euthanized, the retinae were placed on a cellulose membrane soaked overnight in poly-l-lysine (Sigma-Aldrich, St. Louis, MO) and superfused in oxygenated Ames medium (A1420, Sigma-Aldrich, St. Louis, MO) containing sodium bicarbonate (Sigma-Aldrich, St. Louis, MO) at 34 °C, with the RGC side gently pressed against a 60 *µ*m electrode spacing multi-electrode array chip (256MEA60/10iR, Multi Channel Systems). In certain experiments, we perfused the tissue with L-AP4 (50 µM) for at least 20 minutes before the recordings, in order to block input from photoreceptors to ON bipolar cells. Full field light stimuli were presented using a Polychrome V monochromator (TILL Photonics, Munich, Germany) and driven by a STG2008 stimulus generator (Multichannel Systems), using custom written stimuli in MC_Stimulus II (MC_Stimulus II Version 3.4.4, Multi Channel Systems). Output light intensities were calibrated using a spectrophotometer (USB2000+, Oceanoptics, Dunedin, FL). The retinae were stimulated with a light wavelength of 580 nm for a duration of 2 s per presentation at ~10¹⁷ photons cm⁻²s⁻¹. RGC activity was then amplified and sampled at 20 kHz. Signals were filtered with a 200 Hz high pass filter in MC_Rack and individual channels were spike sorted by visualizing the waveforms' principal component analysis plots using Spike2 software v.7 (Cambridge Electronic Design Ltd., Cambridge, UK). Custom-written MATLAB scripts were used for plotting the responses.

By obtaining responses from RGCs following light stimulation, we show that halorhodopsin-triggered responses from transplanted photoreceptors are transmitted to the RGC layer. Figure 4 represents averaged spike responses obtained from MEA recordings shown as PSTH (peristimulus time histogram) and raster plots recorded in NpHR-transplanted Cpfl1/Rho-/- mice (A, B), control GFP-only transplanted Cpfl1/Rho-/- mice (C) and rd1 mice (D). The retinae were stimulated at light wavelength of 580 nm and intensity of 1.24 × 10¹⁷ photons cm⁻² s⁻¹. Figures 4A (for Cpfl1/Rho-/- mouse) and 4D (for rd1 mouse) show representative traces from three RGCs responding either with an ON-response (left), OFF-response (middle) or an ON/OFF-response (right). We were able to obtain three main types of responses that are normally found in RGCs. We later perfused the retina with L-AP4, a blocker of the ON bipolar cell response. This blocked the RGC responses, showing that the responses really do originate from the photoreceptor layer (Figure 4B). The responses reappear after wash-out. As a control, we transplanted blind Cpfl1/Rho-/- mice with GFP-only expressing photoreceptor precursors (Figure 4C). The cells to be transplanted have been prepared and isolated in the same manner as the NpHR-expressing cells, the only difference being that AAV-Rho-GFP has been used for injection in P2 wt mice instead of the AAV-Rho-NpHR-YFP. The fact that no responses have been detected in these mice show us that the photoreceptor precursors alone, without the expression of the optogenetic protein, are not functional or capable of transmitting the signal to the retinal output neurons.

### 8. Light induced behaviour

For the light/dark box experiments, a custom-made light/dark box was constructed (similar to Bourin M, Hascoet M, Eur J Pharmacol. 2003 Feb 28;463(1-3):55-65) but adapted to perform optogenetic stimulation at 590 nm). As shown in Figure 5A, a box of dimensions, 36 cm (I) × 20 cm (b) × 18 cm (h) was divided lengthwise into two equal sized compartments using a non-transparent wall with a 7 cm (b) × 5 cm (h) hole in the middle. The light compartment was fitted with eight 590 nm LEDs (Cree XP-E, amber, Lumitronix), on an aluminum heat sink, at a height of 3 cm from the floor of the cage. All mice were age-matched and their ages ranged between 10-13 weeks at the time of testing. Non-transplanted *Cpfl1*/*Rho*^{*-*/*-*} (n = 10), *Cpfl1*/*Rho*^{*-*/*-*} transplanted with GFP-only expressing cells (n=9), *Cpfl1*/*Rho*^{*-*/-} transplanted with NpHR-expressing cells (N=12),and non-transplanted WT C57BL6J mice (n = 10), were subjected to 4 min trials during which the compartment was illuminated with orange (590 nm) light. The mice were habituated to the testing room in dark for 2 h prior to testing. The behavior of mice was tested at a light intensity of 2.11 × 10¹⁵ photons cm⁻² s⁻¹. Light intensity in the light compartment was adjusted using an adjustable voltage supply (VLP-1303 PRO, Voltcraft) and was measured using a spectrophotometer (OceanOptics). Light intensity measurements were averaged from three locations in the light compartment, 1 cm from the LED, at the center of the cage and immediately next to the hole on the illuminated side. Observers blind to the experimental procedures and treatment groups analyzed the behavior of the mice manually. Mice were introduced individually in the light compartment and allowed to freely explore the box for 4 minutes. The position of the mouse's head was used to define the compartment that it occupied. The time spent in each compartment was recorded by a video camera (Handycam, Sony Corporation, Tokyo, Japan). If an animal never crossed the barrier in the first 3 min in the dark, it was excluded from the trial. Statistical significance was assessed by an ordinary one-way ANOVA and the Tukey's multiple comparisons test was used to compare group means, α level of P < 0.05 was considered significant. Data were analyzed and plotted in Prism 6 (Graphpad Software, La Jolla, CA).

As shown in Figure 5B, both wild type as well as *Cpfl1*/*Rho-*/*-* mice transplanted with NpHR-expressing cells spent significantly less time in the light compartment compared to the non-transplanted *Cpfl1*/*Rho-*/*-* mice or *Cpfl1*/*Rho-*/*-* mice transplanted with GFP-only expressing cells (P < 0.0001, ordinary one-way ANOVA). The difference between wt and NpHR-treated *Cpfl1*/*Rho-*/*-* mice behavior was not significant, as well as the difference in behavior of non-transplanted and GFP-only treated *Cpfl1*/*Rho-*/*-* mice. As light/dark box experiment shows significant light avoidance behavior in NpHR-cell-transplanted mice, it demonstrates that halorhodopsin-induced signals originating from the retina are transmitted to the brain and are sufficient to modulate the behavior of blind mice. Transplanted cells alone (GFP-only, not expressing NpHR) are not enough to trigger a change in behavior.

B. Optogenetic stimulation triggers Halorhodopsin-induced light responses in transplanted photoreceptors of blind *rd10* mice.

Optogenetic transformation of donor (photoreceptor precursor) cells, isolation and transplantation into the blind rd10 mice are performed as described above as patch clamp recording and light stimulation analysis.

Halorhodopsin-expressing cells display a typical light response to two consecutives flashes of light at 590 nm (Figure 6A), photocurrents with spectral tunning with a maximal response obtained at 550-575 nm (Figure 6B and 6C) and typical flicker stimulation responses displaying fast kinetics (Figure 6D).

Live imaging of GFP-positive cells is performed as described above. Halorhodopsin-transduced progenitor cells survived after transplantation in the host and fluorescence was restricted to the cell membrane (Figure 7A: epifluorescence image and B: 2-photon-imaging). Some transplanted cells displayed nice prolongations (Figure 7 A and B, right, arrowheads).

C. Optogenetic stimulation triggers Halorhodopsin-induced light responses in donor cells in whole-mount retina.

At postnatal day P2, C57BL/6J mice were anesthetized on ice, and a cut through the eyelid was made and 1 µl stock containing 3,0 × 10¹³- 3,3 × 10¹⁴ particles of AAV encoding halorhodopsin under the human rhodopsin promoter (AAV9 2YF-hRho-eNpHR-EYFP) was injected into each eye's vitreous cavity using a ultrafine 34-gauge syringe (Hamilton). Retinas were isolated from the C57BL/6J previously injected mice at postnatal day P4. Patch clamp recording and light stimulation analysis with retina are performed as described above.

Halorhodopsin-expressing cells display a typical light response to two consecutives flashes of light at 590 nm (Figure 8A), photocurrents and membrane hyperpolarization with spectral tuning with a maximal response obtained at 575 nm (Figure 8B) and typical flicker stimulation responses with fast kinetics (Figure 8C).

Live imaging of GFP-positive cells is performed as described above. Halorhodopsin-transduced progenitor cells are present 20 days after injection in a whole-mount retina and fluorescence was restricted to the cell membrane (Figure 9A and B).

D. Optogenetic stimulation triggers light responses in AAV-transduced hiPS cells expressing Jaws in retinal organoids.

### 1. Generation of Jaws positive photoreceptors

hiPS cells are produced by transfecting an adult human dermal fibroblast primary cell line with three plasmids coding for the transcription factors *OCT4, SOX2, KLF4 and C-MYC* and cultivated on feeders (Yu J et al. Science, 2009). hiPS cells are expanded to confluence in iPS medium (Essential^{™} 8 medium, GIBCO, Life Technologies). At the confluence, the time status is noted as Day 0. At D0, hiPS cells are placed in iPS medium without FGF-2. At D3, medium is removed and fresh ProN2 medium (Essential 6^{™} medium supplemented with 1% N2 supplement (GIBCO, Life Technologies) is added. On D28, identified neural retinal (NR)-like structures are isolated with a needle, transferred and cultured as a floating structure in maturation medium comprising DMEM/Nutrient Mixture F-12, 1% MEM nonessential amino acids, 2% B27supplement (all from Life Technologies)(also named ProB27 medium) supplemented with 10ng/mL FGF2 to favor neuroretinal development. At day 35, FGF2 was removed. DAPT was added from day 42 to day 49 of differentiation.

### 2. Light stimulation of infected retinal organoids

Retinal organoid were infected on day 70 with 5,0 × 10¹⁰ particles of AAV encoding Jaws under the mCAR (cone arrestin) promoter (AAV2-7m8-mCAR-Jaws-GFP) (Figure 1, bottom). Patch clamp recording and light stimulation analysis with infected retinal organoid are performed as described above.

Jaws-expressing cells display a typical light response while stimulated with two consecutives flashes of light at 590 nm (Figure 10A), hyperpolarization and photocurrents with spectral tuning with a maximal response obtained at 575 nm (Figure 10B), and typical flicker stimulation responses with fast kinetics (Figure 10C).

### 3. Live two-photon-imaging of infected retinal organoids

Live imaging of GFP-positive cells is performed as described above. Live imaging of GFP-positive cells in a retinal organoid at different magnifications indicates that Jaws expression is restricted to PR-membranes (Figure 11).

### 4. Immunofluorescence of D100 monolayer cultures obtained from dissociation of day 70 retinal organoid

### - Retinal organoid dissociation

After removal of any pigmented tissue, 70-day old retinal organoids were collected and washed 3 times in Ringer solution (NaCl 155 mM; KCI 5 mM; CaCl₂ 2 mM; MgCl₂ 1 mM; NaH₂PO₄ 2 mM; HEPES 10 mM and Glucose 10 mM) before dissociation with two units of pre-activated papain at 28.7 u/mg (Worthington) in Ringer solution during 25 min at 37°C. Once a homogeneous cell suspension was obtained after pipetting up and down, papain was deactivated with ProB27 medium. Cells were centrifuged and resuspended in pre-warmed ProB27 medium.

### - Monolayer culture of photoreceptors derived from human iPSC

Dissociated retinal cells were plated on coverslips coated with human recombinant 30 µg/cm² Laminin (Sigma-Aldrich) and 150 µg/cm² Poly-L-Ornithine in 24 well-plates. Monolayers were incubated at 37°C in a standard 5% CO₂ / 95% air incubator and medium was changed every 2 days for the next 15-20 days, before immunostaining.

Cells were washed in PBS (5 min, r.t.) and then permeabilized in PBS containing 0.5 % TRITON^{®} X-100 during 1 hour at r.t. Blocking was done with PBS containing 0.2% gelatin, 0.25% Triton X-100 for 30 min at r.t and incubation with primary antibodies was performed overnight at 4ºC. Primary antibodies used are anti-RECOVERIN rabbit polyclonal antibodies (Millipore) at 1/2000 dilution as photoreceptor marker and anti-GFP chicken polyclonal antibodies (abcam) at 1/500 dilution.

After incubation with primary antibodies, sections were washed with PBS containing 0.25% Tween20 and incubated with Fluorochrome-conjugated secondary antibodies (1/500 dilution) for 1 hour at room temperature. After successive washing in PBS-Tween20, nuclei were counterstained with DAPI (4'-6'-diamino-2-phenylindole, dilactate; Invitrogen-Molecular Probe, Eugene, OR) at a 1/2000 dilution.

As shown in Figure 12, Day 100 monolayers cultures obtained from dissociation of 70 days old Jaws infected retinal organoids present Jaws positive photoreceptor (RCVN) cells.

### 5. Subretinal transplantation of Jaws positive photoreceptor

Infected retinal organoids are dissociated before transplantation. In order to transplant the cell suspension into the subretinal space, adult rd10, Cpfl1/rho^{-/-} or rd1^{-/-} mice were anesthetized by an intraperitoneal injection of ketamine (50mg/kg) and xyazine (10mg/kg). Pupils were dilated by drops of tropicamide (Mydriaticum Dispersa), and by using a syringe with a blunt 34-gauge needle (Hamilton), one microliter suspension containing ~200,000 cells was injected into the subretinal space (Figure 1, bottom).

### 6. Transplanted Jaws positive cells (GFP) in Rd10 mice are integrated in the outer nuclear layer and expressed photoreceptor specific markers.

### Tissue preparation

100 days old transplanted rd10 mice eyes were enucleated and immediately fixed overnight at 4ºC in freshly prepared 4% paraformaldehyde solution. Eyes were washed in PBS and incubated overnight in 30% sucrose in PBS solution before inclusion in OCT.

18um thick sections from the eye cups were obtained using a Cryostat Microm and mounted on Super Frost Ultra Plus^{®} slides (MENZEL-GLASER, Braunschweig, Germany). Cryosections were washed in PBS (5 min, r.t.) to remove the rest of OCT and then permeabilised in PBS containing 0.5 % TRITON^{®} X-100 during 1 hour at r.t. Blocking was done with PBS containing 0.2% gelatin, 0.25% Triton X-100 for 30 min at r.t and incubation with primary antibodies was performed overnight at 4ºC. Primary antibodies used are anti-CRX mouse monoclonal antibodies (Abnova) at 1/5000 dilution and anti-RCVN rabbit polyclonal antibodies (Millipore) at 1/2000 dilution as photoreceptor markers; anti-R/G opsin rabbit polyclonal antibodies (Millipore) at 1/200 dilution as cone specific marker; anti-PDE6C rabbit polyclonal antibodies at 1/200 dilution; anti-GFP chicken polyclonal antibodies (abcam) at 1/500 dilution and anti-RIBEYE mouse monoclonal antibodies (BD Biosciences) at 1/500 dilution.

After incubation with primary antibodies, sections were washed with PBS containing 0.25% Tween20 and incubated with Fluorochrome-conjugated secondary antibodies (1/500 dilution) for 1 hour at room temperature. After successive washing in PBS-Tween20, nuclei were counterstained with DAPI (4'-6'-diamino-2-phenylindole, dilactate; Invitrogen-Molecular Probe, Eugene, OR) at a 1/2000 dilution. Samples were further washed in PBS and dehydrated with 100% ethanol before mounting using fluoromount Vectashield (Vector Laboratories).

Immunofluorescence analysis on retinal sections from 100 days old transplanted rd10 mice show Jaws positive cells (GFP) integrated in the ONLand expressed photoreceptor specific markers. Positive cells for Jaws and CRX and RCVN as well as cone specific marker R/G OPSIN. No PDE6C (cone specific) was observed in donor or host cells (Figure 13A). Jaws positive photoreceptor cells connect with bipolar cells and form synaptic connections (Figure 13B).

Thus, Jaws positive cells (GFP) integrated in the ONL and expressed photoreceptor specific markers. Jaws positive cells made synaptic connections with the subjacent bipolar cells

### 7. Light induced behaviour of transplanted Jaws positive cells in Rd10 mice.

As shown in Figure 14, Light behavioral analysis were performed using the Light/Dark box test on 90 days old rd10 mice. Figure 14 represents the time spent in light box (%) under the light condition by non transplanted blind rd10 mice (n=12), single-eye transplanted rd10 mice (n=10), rd10 mice transplanted in both eyes (n=3). Statistical significance was assessed performing a Mann-Whitney U test to compare group means. A p value of P < 0,05 was considered significant. Data were analyzed and plotted in Prism 6 (Graphpad Software, La Jolla, CA). ^{∗}: p =0,0070 and ^{∗∗}: p = 0,0091.

Thus, these results show that transplanted mice exhibit light avoidance behavior compared to the rd10 blind mice control when both eyes were injected.

### 8. Light stimulation of transplanted Jaws positive cells in Cpfl1/Rho^{-/}⁻ and Rd1 model.

Patch clamp recording and light stimulation analysis were performed as described above.

Photocurrents (top) and voltage hyperpolarization (bottom) were analyzed from cells expressing Jaws stimulated with 2 consecutive flashes of 590 nm light at an intensity of 3.5 × 10¹⁷ photons cm⁻² s⁻¹ in *Cpfl1*/*Rho*^{*-*/*-*} (Figure 15A) and *rd1* model (Figure 15 D). Figures 15B and F represent Jaws-induced hyperpolarization action spectrum corresponding to a Jaws-expressing cell stimulated at different wavelengths. Stimuli ranging from 400 nm to 650 nm, separated by 25 nm steps, were used at 3.5 × 10¹⁷ photons cm⁻² s⁻¹. Maximal responses were obtained at 575 nm in both Cpfl1/Rho-/- (Figure 15B) and rd1 model (Figure 15 F), which corresponds to the action spectrum maximum of Jaws Temporal properties were then analyzed by modulation of Jaws-induced hyperpolarization at increasing stimulation frequencies in transplanted Cpfl1/Rho-/- mouse (Figure 15 C) and rd1 mouse (Figure 15 G), from 2 to 30 Hz (at 3.5 × 1017 photons cm-2 s-1). A magnified trace is shown for 25 Hz in Cpfl1/Rho-/- model (Figure 15C). This is a sufficient temporal resolution for human vision. Figure 15E represents Jaws voltage-responses as a function of light stimulation intensity (from 1014 to 1017 photons cm-2 s-1), showing that lower light intensities are also sufficient to trigger a response in transplanted cells, although to a lower extent.

### 9. Transfer of the signal from hiPS to ganglion cells through interneurons.

In order to show an example of the signal transfer from our transplanted hiPSC-derived cells (input of the retina) to the ganglion cells (output of the retina), we managed to record an interneuron (a second order neuron that doesn't express Jaws) located just below 3 transplanted fluorescent cells, since it had a high probability to be directly connected to them. A schematic view of this signal transfer is represented on Figure 16. Representative photocurrents from a cell expressing Jaws stimulated with 2 consecutive flashes are represented on Figure 16A, followed by representative currents (bottom) and voltage (top) responses from a second order cell (OFF cell) in the INL layer that was not expressing Jaws but was very likely connected to surroundings Jaws-hiPS transplanted cells (Figure 16B) and finally an example of an OFF-ganglion cell (third order neuron) response (spiking activity) recording that could hypothetically receive its input from the second order neuron displayed in Figure 16B (Figure 16C).

### 10. Multi-electrode array recordings and data analysis

Multi-electrode array recordings, light stimulation and data analysis were performed as described above.

Figure 17A represents averaged spike responses from MEA recordings shown as PSTH and raster plots from transplanted blind Cpfl1/Rho^{-/-}mouse. The responses were triggered with 2 second light pulses of 580nm light of intensity 1.24× 10¹⁷ photons cm⁻² s⁻¹. RGCs responding with either an OFF (left) or an ON/OFF (right) response have been detected. We also recorded ON and OFF responses in transplanted rd1 mice - representative examples are shown in Figure 17F. We further stimulated transplanted Cpfl1/Rho^{-/-} retina with wavelengths ranging from 450 to 650 nm (intensity 1.24× 10¹⁷ photons cm⁻² s⁻¹). RGCs showed no response when exposed to 450 nm stimuli, corresponding to the fact that the action spectrum of Jaws is red-shifted (Figure 17B). We also used lower light intensities for stimulation and showed that stimuli of intensity of 10¹⁶ photons cm⁻² s⁻¹ (at 580 nm) were enough to trigger a response in RGCs, although to a lower extent (Figure 17C). Figure 17D represents average spike responses and raster plots from a representative RGC that were the results of 1 s (left), 100 ms (middle) and 1 ms (right) stimulations (580nm, 1.24x 10¹⁷ photons cm⁻² s⁻¹). Stimuli as short as 1 ms are enough to trigger a robust response. Figure 17E shows a representative example recorded from a retina that has been treated with GFP-only expressing iPSC-derived photoreceptors. The preparation and isolation of these cells have been done in the same manner as for the Jaw-expressing cells, the only difference being that an AAV-mCar-GFP virus has been added to the medium instead of the AAV-mCar-Jaws-GFP. The results show us that iPSC-derived photoreceptors alone, without Jaws expression, are not enough to restore responses in RGCs.

## Claims

1. An isolated photoreceptor precursor cell comprising a heterologous nucleic acid encoding an optogenetic inhibitor, wherein said inhibitor is selected from the group consisting of halorhodopsins, archaerhodopsin-3 (AR-3), archaerhodopsin (Arch), bacteriorhodopsins, proteorhodopsins, xanthorhodopsins, Leptosphaeria maculans fungal opsins (Mac) and Jaws.

2. The photoreceptor precursor cell of claim 1, wherein said heterologous nucleic acid is operably linked to a specific-photoreceptor promoter.

3. The photoreceptor precursor cell of claim 1 or 2, wherein said heterologous nucleic acid is contained in a recombinant viral vector, preferably an adeno-associated virus.

4. The photoreceptor precursor cells of any one of claims 1 to 3, wherein said optogenetic inhibitor is selected from halorhodopsins and Jaws.

5. The photoreceptor precursor cell according to any one of claims 1 to 4, wherein said photoreceptor precursor cell was obtained from differentiation of stem cells, preferably induced pluripotent stem cells.

6. A pharmaceutical composition comprising a photoreceptor precursor cell according to any one of claims 1 to 5 and a pharmaceutically acceptable excipient.

7. The pharmaceutical composition according to claim 6, wherein said composition is formulated for intraocular injection.

8. Photoreceptor precursor cell according to any one of claims 1 to 5 or pharmaceutical composition of claim 6 or 7 for use in the treatment of a retinal degenerative disease.

9. The photoreceptor precursor cell or pharmaceutical composition for use of claim 8, wherein said retinal degenerative disease is related to a loss of function or death of photoreceptor.

10. The photoreceptor precursor cell or pharmaceutical composition for use of claim 8 or 9, wherein said cell or composition is to be administered to the subject in need thereof by intraocular injection, preferably by injection into the subretinal space of the eye.

11. The photoreceptor precursor cell or pharmaceutical composition for use of any one of claims 8 to 10, wherein said retinal degenerative disease is selected from the group consisting of macular degeneration, retinitis pigmentosa, cone dystrophy, Usher syndrome, rod dystrophy, rod-cone dystrophy, achromatopsia and Bardet-Biedl syndrome.

12. The photoreceptor precursor cell or pharmaceutical composition for use of any one of claims 8 to 11, wherein said disease is retinitis pigmentosa.

13. The photoreceptor precursor cell or pharmaceutical composition for use according to any one of claims 8 to 12 in patient with advanced stage of photoreceptor degeneration.

14. An *in vitro* method for producing a photoreceptor precursor cell of any one of claims 1 to 5, comprising introducing a nucleic acid encoding an optogenetic inhibitor into a provided photoreceptor precursor cell, wherein the nucleic acid and the optogenic inhibitor are as defined in any one of claims 1 to 5.

15. The method of claim 14, wherein the photoreceptor precursor cell was obtained from differentiation of induced pluripotent stem cells, said stem cells being obtained from somatic cells from a patient suffering from a retinal generative disease.

## Patentansprüche

1. Isolierte Photorezeptor-Vorläuferzelle, umfassend eine heterologe Nukleinsäure, die für einen optogenetischen Inhibitor kodiert, wobei der Inhibitor ausgewählt ist aus der Gruppe bestehend aus Halorhodopsinen, Archaerhodopsin-3 (AR-3), Archaerhodopsin (Arch), Bakteriorhodopsinen, Proteorhodopsinen, Xanthorhodopsinen, Leptosphaeria maculans fungal opsins (Mac) und Jaws.

2. Photorezeptor-Vorläuferzelle nach Anspruch 1, wobei diese heterologe Nukleinsäure funktionsfähig mit einem spezifischen Photorezeptor-Promotor verbunden ist.

3. Photorezeptor-Vorläuferzelle nach Anspruch 1 oder 2, wobei diese heterologe Nukleinsäure in einem rekombinanten viralen Vektor, vorzugsweise einem Adeno-assoziierten Virus, enthalten ist.

4. Photorezeptor-Vorläuferzellen nach einem der Ansprüche 1 bis 3, wobei der optogenetische Inhibitor ausgewählt ist aus Halorhodopsinen und Jaws.

5. Photorezeptor-Vorläuferzelle nach einem der Ansprüche 1 bis 4, wobei diese Photorezeptor-Vorläuferzelle durch Differenzierung von Stammzellen, vorzugsweise induzierten pluripotenten Stammzellen, erhalten war.

6. Pharmazeutische Zusammensetzung, umfassend eine Photorezeptor-Vorläuferzelle nach einem der Ansprüche 1 bis 5 und einen pharmazeutisch verträglichen Hilfsstoff.

7. Pharmazeutische Zusammensetzung nach Anspruch 6, wobei die Zusammensetzung zur intraokularen Injektion formuliert ist.

8. Photorezeptor-Vorläuferzelle nach einem der Ansprüche 1 bis 5 oder pharmazeutische Zusammensetzung nach Anspruch 6 oder 7 zur Verwendung bei der Behandlung einer retinalen degenerativen Erkrankung.

9. Photorezeptor-Vorläuferzelle oder pharmazeutische Zusammensetzung zur Verwendung nach Anspruch 8, wobei diese retinale degenerative Erkrankung mit einem Funktionsverlust oder Tod des Photorezeptors zusammenhängt.

10. Photorezeptor-Vorläuferzelle oder pharmazeutische Zusammensetzung zur Verwendung nach Anspruch 8 oder 9, wobei diese Zelle oder Zusammensetzung dem Subjekt, das sie benötigt, durch intraokulare Injektion, vorzugsweise durch Injektion in den sub-retinalen Raum des Auges, zu verabreichen ist.

11. Photorezeptor-Vorläuferzelle oder pharmazeutische Zusammensetzung zur Verwendung nach einem der Ansprüche 8 bis 10, wobei diese retinale degenerative Erkrankung ausgewählt ist aus der Gruppe bestehend aus Makuladegeneration, Retinitis pigmentosa, Zapfendystrophie, Usher-Syndrom, Stäbchendystrophie, Stäbchen-Zapfen Dystrophie, Achromatopsie und Bardet-Biedl-Syndrom.

12. Photorezeptor-Vorläuferzelle oder pharmazeutische Zusammensetzung zur Verwendung nach einem der Ansprüche 8 bis 11, wobei die Krankheit Retinitis pigmentosa ist.

13. Photorezeptor-Vorläuferzelle oder pharmazeutische Zusammensetzung zur Verwendung nach einem der Ansprüche 8 bis 12 bei einem Patienten mit fortgeschrittenem Stadium der Photorezeptor-Degeneration.

14. In-vitro-Verfahren zur Herstellung einer Photorezeptor-Vorläuferzelle nach einem der Ansprüche 1 bis 5, umfassend das Einführen einer Nukleinsäure, die für einen optogenetischen Inhibitor kodiert, in eine bereitgestellte Photorezeptor-Vorläuferzelle, wobei die Nukleinsäure und der optogene Inhibitor sind, wie in einem der Ansprüche 1 bis 5 definiert.

15. Verfahren nach Anspruch 14, wobei die Photorezeptor-Vorläuferzelle aus der Differenzierung von induzierten pluripotenten Stammzellen erhalten war, wobei diese Stammzellen aus somatischen Zellen eines Patienten erhalten waren, der an einer retinalen generativen Erkrankung leidet.

## Revendications

1. Cellule précurseur de photorécepteur isolée comprenant un acide nucléique hétérologue encodant un inhibiteur optogénétique, dans laquelle ledit inhibiteur est sélectionné dans le groupe constitué par les halorhodopsines, l'archaerhodopsine-3 (AR-3), l'archaerhodopsine (Arch), les bactériorhodopsines, les protéorhodopsines, les xanthorhodopsines, les opsines fongiques de Leptosphaeria maculans (Mac) et Jaws.

2. Cellule précurseur de photorécepteur selon la revendication 1, dans laquelle ledit acide nucléique hétérologue est lié de manière opérationnelle à un promoteur de photorécepteur spécifique.

3. Cellule précurseur de photorécepteur selon la revendication 1 ou 2, dans laquelle ledit acide nucléique hétérologue est contenu dans un vecteur viral recombiné, de préférence un virus adéno-associé.

4. Cellule précurseur de photorécepteur selon l'une quelconque des revendications 1 à 3, dans laquelle ledit inhibiteur optogénétique est sélectionné parmi les halorhodopsines et Jaws.

5. Cellule précurseur de photorécepteur selon l'une quelconque des revendications 1 à 4, laquelle cellule précurseur de photorécepteur a été obtenue à partir d'une différenciation de cellules souches, de préférence de cellules souches pluripotentes induites.

6. Composition pharmaceutique comprenant une cellule précurseur de photorécepteur selon l'une quelconque des revendications 1 à 5 et un excipient pharmaceutiquement acceptable.

7. Composition pharmaceutique selon la revendication 6, laquelle composition est formulée pour une injection intraoculaire.

8. Cellule précurseur de photorécepteur selon l'une quelconque des revendications 1 à 5 ou composition pharmaceutique selon la revendication 6 ou 7, pour une utilisation dans le traitement d'une maladie dégénérative rétinienne.

9. Cellule précurseur de photorécepteur ou composition pharmaceutique pour une utilisation selon la revendication 8, dans laquelle ladite maladie dégénérative rétinienne est liée à une perte de fonction ou à la mort d'un photorécepteur.

10. Cellule précurseur de photorécepteur ou composition pharmaceutique pour une utilisation selon la revendication 8 ou 9, laquelle cellule ou composition est destinée à être administrée au sujet en ayant besoin par injection intraoculaire, de préférence par injection dans l'espace sous-rétinien de l'œil.

11. Cellule précurseur de photorécepteur ou composition pharmaceutique pour une utilisation selon l'une quelconque des revendications 8 à 10, dans laquelle ladite maladie dégénérative rétinienne est sélectionnée dans le groupe constitué par une dégénérescence maculaire, une rétinite pigmentaire, une dystrophie des cônes, un syndrome d'Usher, une dystrophie des bâtonnets, une dystrophie des cônes et des bâtonnets, une achromatopsie et un syndrome de Bardet-Biedl.

12. Cellule précurseur de photorécepteur ou composition pharmaceutique pour une utilisation selon l'une quelconque des revendications 8 à 11, dans laquelle ladite maladie est une rétinite pigmentaire.

13. Cellule précurseur de photorécepteur ou composition pharmaceutique pour une utilisation selon l'une quelconque des revendications 8 à 12 chez un patient à un stade avancé de dégénérescence des photorécepteurs.

14. Méthode *in vitro* pour produire une cellule précurseur de photorécepteur de l'une quelconque des revendications 1 à 5, comprenant l'introduction d'un acide nucléique encodant un inhibiteur optogénétique dans une cellule précurseur de photorécepteur fournie, dans laquelle l'acide nucléique et l'inhibiteur optogénétique sont tels que définis dans l'une quelconque des revendications 1 à 5.

15. Méthode selon la revendication 14, dans laquelle la cellule précurseur de photorécepteur a été obtenue à partir d'une différenciation de cellules souches pluripotentes induites, lesdites cellules souches ayant été obtenues à partir de cellules somatiques provenant d'un patient souffrant d'une maladie dégénérative rétinienne.
